# EUROPEAN PATENT SPECIFICATION

(11) **EP 1 943 358 B1**
(45) Date of publication and mention of the grant of the patent: **04.03.2015**
(21) Application number: 06814243.9
(22) Date of filing: 08.09.2006
(51) Int. Cl.: C12Q 1/68

(54) **CYP1B1 GENOTYPE**
CYP1B1-GENOTYP
GENOTYPE CYP1B1

(30) Priority: 12.09.2005 US 716439 P
(43) Date of publication of application: 16.07.2008
(73) Proprietor: Government of the USA, as Represented by the Secretary, Department of Health and Human Services, Rockville, MD 20852 (US)
(72) Inventor: SPARREBOOM, Alex, Baltimore, MD 21224 (US); FIGG, William, D., Fairfax, VA 22030 (US); SISSUNG, Tristan, M., Annandale, VA 22003 (US); PRICE, Douglas, K., Fairfax, VA 22032 (US)
(74) Representative: Bosch, Matthias
(86) International application number: PCT/US2006/034769
(87) International publication number: WO 2007/032984

(56) References cited:
- WO-A-01/58444
- WO-A-02/30951
- WO-A-02/083839
- WO-A-03/025141
- WO-A-03/027640
- SISSUNG TRISTAN M ET AL: "Association of the CYP1B1*3 allele with survival in patients with prostate cancer receiving docetaxel." PHARMACOTHERAPY, vol. 25, no. 10, October 2005 (2005-10), page 1479, XP002427890 ANNUAL MEETING OF THE AMERICAN-COLLEGE-OF-CLINICAL-PHARMACY; SAN FRANCISCO, CA, USA; OCTOBER 23 -26, 2005 ISSN: 0277-0008
- SCRIPTURE ET AL: "Modulation of cytochrome P450 activity: implications for cancer therapy" LANCET ONCOLOGY, LANCET PUBLISHING GROUP, LONDON, GB, vol. 6, no. 10, October 2005 (2005-10), pages 780-789, XP005162199 ISSN: 1470-2045
- THIER R ET AL: "Markers of genetic susceptibility in human environmental hygiene and toxicology: The role of selected CYP, NAT and GST genes" INTERNATIONAL JOURNAL OF HYGIENE AND ENVIRONMENTAL HEALTH, URBAN U. FISCHER, JENA, DE, vol. 206, no. 3, 2003, pages 149-171, XP004960259 ISSN: 1438-4639
- LANDI ET AL: "CYP1A1 and CYP1B1 genotypes, haplotypes, and TCDD-induced gene expression in subjects from Seveso, Italy" TOXICOLOGY, LIMERICK, IR, vol. 207, no. 2, 14 February 2005 (2005-02-14), pages 191-202, XP005004309 ISSN: 0300-483X cited in the application
- SORENSEN M ET AL: "Genetic polymorphisms in CYP1B1, GSTA1, NQO1 and NAT2 and the risk of lung cancer" CANCER LETTERS, NEW YORK, NY, US, vol. 221, no. 2, 28 April 2005 (2005-04-28), pages 185-190, XP004829537 ISSN: 0304-3835 cited in the application
- BRÜNING T ET AL: "Real-time PCR-analysis of the cytochrome P450 1B1 codon 432-polymorphism." ARCHIVES OF TOXICOLOGY NOV 1999, vol. 73, no. 8-9, November 1999 (1999-11), pages 427-430, XP002427186 ISSN: 0340-5761
- HIRATSUKA ET AL: "Genetic testing for pharmacogenetics and its clinical application in drug therapy" CLINICA CHIMICA ACTA, AMSTERDAM, NL, vol. 363, no. 1-2, 26 August 2005 (2005-08-26), pages 177-186, XP005194912 ISSN: 0009-8981
- MURRAY ET AL.: "Cytochrome P450 CYP1B1: A novel mechanism of drug resistance" BRITISH JOURNAL OF CANCER, vol. 85, no. Supplement 1, July 2001 (2001-07), page 19, XP002427185 MEETING OF THE BRITISH JOURNAL OF CANCER RESEARCH; LEEDS, UK; JULY 01-04, 2001 ISSN: 0007-0920
- MCFADYEN MORAG C E ET AL: "Cytochrome P450 CYP1B1: A novel mechanism of drug resistance" BRITISH JOURNAL OF CANCER, vol. 85, no. Supplement 1, July 2001 (2001-07), page 19, XP002427948 MEETING OF THE BRITISH JOURNAL OF CANCER RESEARCH; LEEDS, UK; JULY 01-04, 2001 ISSN: 0007-0920
- MCFADYEN MORAG C E ET AL: "Cytochrome P450 CYP1B1 activity in renal cell carcinoma" BRITISH JOURNAL OF CANCER, vol. 91, no. 5, 31 August 2004 (2004-08-31), pages 966-971, XP002427184 ISSN: 0007-0920
- ROBERT J ET AL: "Predicting drug response and toxicity based on gene polymorphisms" CRITICAL REVIEWS IN ONCOLOGY/HEMATOLOGY, BOCARATON, FL, US, vol. 54, no. 3, June 2005 (2005-06), pages 171-196, XP004884954 ISSN: 1040-8428
- DATABASE EMBL [Online] 14 July 1994 (1994-07-14), "Human dioxin-inducible cytochrome P450 (CYP1B1) mRNA, complete cds." XP002427241 retrieved from EBI accession no. EMBL:U03688 Database accession no. U03688

## Description

### GOVERNMENT SUPPORT

This work described herein was supported by the National Institutes of Health. The U.S. Government may have certain rights in the invention.

### BACKGROUND

Cytochrome P450 is one of the enzymes that participate in the Phase I metabolism of chemical substances, such as active ingredient compounds in medicines. A number of molecular species of cytochrome P450 are known to exist, and about 22 molecular species have been hitherto confirmed in humans. Although these molecular species are different in enzyme activity from one another, they are each reported to participate in the metabolism of active ingredient compounds in medicines (e.g., active ingredient compounds in tricyclic antidepressants, antiepileptics, benzodiazepine preparations, beta-blockers, barbital sleep-inducing hypnotics, and other medicines, dimethylnitrosamine, etc.), benzene and other organic solvents, low-molecular-weight carcinogens in the environment, or the like (for example, Yoo, J. S. H., Chung, R., C., Wade, D., & Yang, C. S. (1987) Cancer Res., 47, 3378-3383).

Mammalian cytochrome P450 genes encode a superfamily of hemeproteins that are active in the oxidative metabolism of endogenous and exogenous compounds. Cytochrome P450 (referred to as CYP) are now classified into families on the basis of amino acid similarity; within families cytochrome P450 exhibit >40% similarity and >55% similarity within subfamilies. The cytochrome P450 enzymes are designated by the letters "CYP" followed by a numeral, a letter and another numeral (e.g. CYP1B1). In humans there are more than 20 different CYP enzymes.
Docetaxel is one of the most frequently prescribed anti-cancer agents. It is derived from the taxane family of agents that stabilize tubulin at the mitotic spindle resulting in cell cycle arrest followed by apoptosis. Despite the relative success of docetaxel in the treatment of various cancers, including androgen-independent prostate cancer (AIPC), high variability in clinical response of docetaxel has been observed (1). This variability can be attributed in part to a poor understanding of inter-subject differences in docetaxel pharmacokinetics and pharmacodynamics. Cellular sensitivity toward docetaxel has been associated with the isozyme cytochrome P450 1B1 (CYP1B1) in previous preclinical studies. CYP1B1 is a heme-thiolate mono-oxygenase that is involved in the mono-oxygenation of a variety of substrates, including steroids and xenobiotics. Docetaxel competitively inhibits CYP1B1-mediated ethoxyresorufin O-deethylation and CYP1B1 overexpression results in docetaxel resistance in transfected Chinese hamster ovary cells, an effect that is reversible by chemical inhibition (2, 3). CYP1B1 overexpression has also been implicated in premalignant progression and carcinogenesis of many hormone-mediated malignancies, including prostate (4), ovarian (5), and breast cancers (6). Furthermore, CYP1B1 is upregulated within tumor cells as compared to surrounding normal tissue, and has been attributed to alterations in tumor metabolism that could contribute to tumorogenesis, and altered drug metabolism (7, 20). The CYP1B1 gene is highly polymorphic and several non-synonymous single nucleotide polymorphisms contained within the CYP1B1 gene have been identified that alter the expression and/or activity of the encoded protein. Of these, the CYP1B1*3 (4326C>G; L432V) allele is characterized by both increased expression and enzyme kinetics of CYP1B1 toward several substrates (8-11). Based on these observations, we hypothesized that the CYP1B1 genotype could modulate the therapeutic response to docetaxel treatment.

Given the clinical importance of docetaxel, genetic markers with predictive power to assess inter-subject differences resulting in clinical outcome prior to docetaxel administration are urgently needed.

### SUMMARY

Provided herein are identifications of genes and sequence variances which can be useful in connection with predicting differences in response to treatment and selection of appropriate treatment of a disease or condition.

These variances may be useful either during the drug development process or in guiding the optimal use of already approved compounds. DNA sequence variances in candidate genes (e.g., genes that may plausibly affect the action of a drug) areanalyzed, leading to the establishment of diagnostic tests useful for improving the development of new pharmaceutical products and/or the more effective use of existing pharmaceutical products. Methods for identifying genetic variances and determining their utility in the selection of optimal therapy for specific patients are also described. In general, the invention relates to methods for identifying and dealing effectively with the genetic sources of interpatient variation in drug response, including variable efficacy as determined by pharmacokinetic variability.

Also, described herein is the identification of gene sequence variances in CYP1B1 that are predictive of drug action and are useful for determining drug efficacy in an subject subject.

In one aspect, provided herein are methods of predicting responsiveness of a tumor to therapeutic treatment comprising determining a CYP1B1 genotype status of a tumor cell, and correlating the genotype status to the therapeutic treatment.

In one embodiment, the genotype status is determined by PCR methods, immunological methods, sequencing methods, expression level of CYP1B1, enzyme kinetics of CYP1B1.

In another embodiment, the CYP1B1 genotype status at nucleotide position 4326 is determined. In a related embodiment, the CYP1B1 genotype status at nucleotide position 4326 is determined by one or more of sequencing methods, PCR methods, SNP Chip technology, or RFLP. In another related embodiment, PCR methods are one or more of real-time PCR, PCR, reverse transcriptase PCR, or allele-specific PCR.

According to one embodiment, the CYP1B1 genotype status of at amino acid position 432 is determined. In a related embodiment, the CYP1B1 genotype status at amino acid position 432 is determined by one or more of immunological methods or sequencing methods.

In one embodiment, a wild-type genotype status or heterozygous genotype status correlates with responsiveness of a tumor to therapeutic treatment. In a related embodiment, the wildtype genotype status is L at amino acid position 432 and C at nucleotide position 4236.

In another embodiment, a homozygous variant CYP1B1 genotype status correlates with unresponsiveness of a tumor to therapeutic treatment. In a related embodiment, the homozygous variant CYP1B1 genotype status is V at amino acid position 432 and G at nucleotide position 4236.

According to one embodiment, the methods provided herein may further comprise administering a therapeutic amount of an anti-neoplastic agent to the subject. In a related embodiment, the anti-neoplastic agent comprises docetaxel. In another related embodiment, the methods may further comprise co-administering one or more additional anti-neoplastic agents to the subject. In another related embodiment, the one or more additional anti-neoplastic agents are selected from cisplatin, cyclophosphamind, doxorubicin, prednisone, 5-FU, trastuzumab, 3G4, travacin, gemcitabine, estramustine, carboplatin, or radioimmunotherapy agents. In another related embodiment, the methods may further comprise co-administering one or more additional therapeutic agents to the subject. In a related embodiment, the therapeutic agents are one or more of an immunomodulatory agent, anti-inflammatory agents, glucocorticoid, steroid, non-steriodal anti-inflammatory drug, leukotreine antagonist, β2-agonist, anticholinergic agent, sulphasalazine, penicillamine, dapsone, antihistamines, anti-malarial agents, anti-viral agents, or antibiotics. In another related embodiment, the additional therapeutic agent is prednisone.

In one embodiment, the tumor is one or more of breast, prostate, lung, head and neck, mesothelioma, ovarian, urothelial, hepatocellular, bladder, esopheageal, or stomach.

In one aspect, provided herein are methods of selecting a subject that will respond to docetaxel treatment, comprising detecting the presence or absence of a variation at nucleotide position 4326 or amino acid position 432 of CYP1B1, and correlating an absence of a variation or heterozygous variation with an indication that the subject will respond to docetaxel treatment.

In another embodiment, the methods may further comprise correlating the presence of a variation with an indication that the subject will not respond to docetaxel treatment. In another related embodiment, the methods may further comprise administering a therapeutic amount of an anti-neoplastic agent to a subject having the absence of a variation or heterozygous variation.

In one aspect, provided herein are methods of assessing the risk of cancer in a subject, comprising determining the genotype status of CYP1B1, and correlating the genotype status to cancer risk.

In one aspect, provided herein are methods of assessing the responsiveness of a subject to treatment with a tubulin stabilization agent, comprising determining a CYP1B1 genotype status of a subject or a cell of a subject, and correlating the genotype status to the efficacy of the tubulin stabilization agent.

In one embodiment, the tubulin stabilization agent is selected from one or more of docetaxel, paclitaxel, and derivatives thereof.

In one aspect, provided herein are kits for the assessment of cancer treatment options, comprising oligonucleotide probes that differentiate the wild-type and variant alleles of CYP1B1 and instructions for use, wherein the allele nucleotide position 4326.

In one embodiment, the oligonucleotide probes are one or more of OLA or Taqman probes.

In one aspect, provided herein are kits for the assessment of cancer treatment options, comprising oligonucleotide primes that amplify from about nucleotide 4300 to about nucleotide 4350 portion of CYP1B1 and instructions for use.

In one aspect, provided herein are kits for the assessment of cancer treatment options, comprising a microarray, at least one oligonucleotide primer that amplifies from about nucleotide 4300 to about nucleotide 4350 of CYP1B1 and instructions for use.

In one aspect, provided herein are methods for determining the therapeutic capacity of a tubulin stabilization agent to reduce tissue degeneration in a subject, comprising determining a CYP1B1 genotype status of a subject or a cell of a subject; determining a pre-treatment tumor status in the subject; administering a therapeutically effective amount of a tubulin stabilization agent to the subject; and determining a post-treatment tumor status in the subject.

In one embodiment, a modulation of tumor status indicates that the tubulin stabilization agent is efficacious. In another embodiment, the pre-treatment and post-treatment levels of tumor status are determined in a diseased tissue. In a related embodiment, the diseased tissue is one or more of a fetus, lung, heart, liver, breast, prostate, vasculature or nervous tissue. In one aspect, provided herein are methods for determining the therapeutic capacity of a candidate tubulin stabilization agent for treating cancer, comprising providing a population of tumor cells with a known CYP1B1 genotype status; contacting the cells with a candidate composition, and determining effect of the candidate composition on cell proliferation, wherein a decrease in cell proliferation indicates that the candidate composition may be efficacious.

In one embodiment, the methods may further comprise correlating the effect with the genotype.

In one embodiment, the methods may further comprise determining the CYP1B1 genotype status of the tumor cells prior to or after providing the cells.

In one aspect, provided herein are methods of treating a subject suffering from cancer, comprising determining a CYP1B1 genotype status of a subject or a cell of a subject, and administering a therapeutic amount of a tubulin stabilization agent to a heterozygous or a wild-type subject.

In one embodiment, the subject is a mammal. In a related embodiment, the mammal is a human.

In one embodiment, the tubulin stabilization agent comprises docetaxel.

In one embodiment, the methods may further comprise co-administering one or more additional therapeutic agents to the heterozygous or wild-type subject.

Other embodiments of the invention are disclosed *infra.*

### BRIEF DESCRIPTION OF THE DRAWING

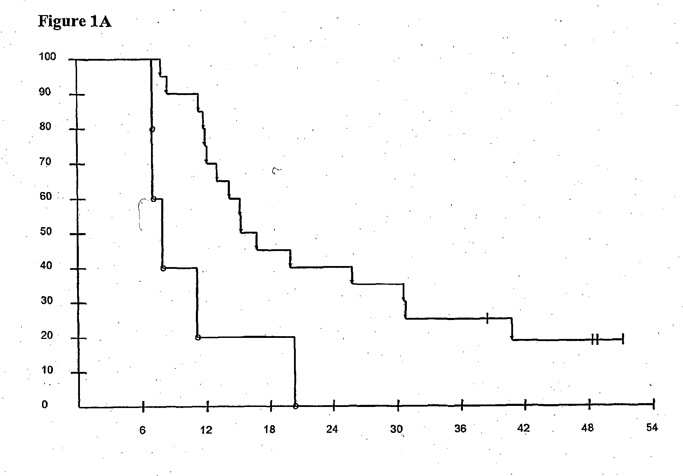

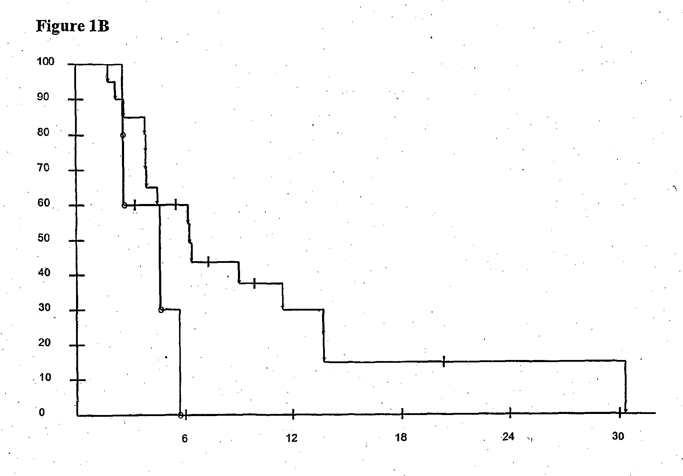

Figure 1 depicts Kaplan-Meier curves of overall survival (o - CYP1B1*3; * - CYP1B1*1/*1 and CYP1B1*1/*3) (A) and overall progression free survival (B) in men with prostate cancer treated with docetaxel as a function of CYP1B1*3 genotype. All patients received docetaxel as a 1-hour intravenous infusion at a dose of 30 mg/m² in cycles of once every week for 3 consecutive weeks, followed by a 1-week rest period (n = 25). DNA was extracted from a plasma samples using a QiaBlood extraction kit (Qiagen, Valencia, CA) and stored at 4°C. The CYP1B1*3 alleles were analyzed by direct sequencing. The primers used for sequencing and amplification of the variant were as follows: F4 - GGTATCCTGATGTGCAGACTCG; R4 - TGGACAGCACTATCAAGGAGCT; F5 - TGCCTGTCACTATTCCTCATGCC; R5 - GGTGAGCCAGGATGGAGATGA. A 50-µl reaction was prepared for polymerase chain reaction (PCR) amplification using primers F4 and R4. The reaction consisted of 1 × PCR buffer, 2 mM of each of the four deoxynucleotidetriphosphates (dNTPs), 1.5 mM magnesium chloride, and 1 unit of Platinum Taq DNA polymerase. PCR conditions were: 94° C for 5 minutes, followed by 40 cycles of 94° C for 30 seconds, 66° C for 30 seconds, and 72° C for 30 seconds, with a final 7-minute cycle at 72° C. Direct nucleotide sequencing PCR was conducted using the Big Dye Terminator Cycle Sequencing Ready Reaction kit V1.1 on an ABI Prism 310 Genetic Analyzer (Applied BioSystems, Foster City, CA).

### DETAILED DESCRIPTION

Disclosed herein is a target gene and variances having utility in pharmacogenetic association studies and diagnostic tests to improve the use of certain drugs or other therapies including, for example, docetaxel and other tubulin stabilization agents that may be described in the 1999 Physicians' Desk Reference (53rd edition), Medical Economics Data, 1998, or the 1995 United States Pharmacopeia XXIII National Formulary XVIII, Interpharm Press, 1994, or other sources as described below.

### Definitions

As used herein, the term "polymorphic site" refers to a region in a nucleic acid at which two or more alternative nucleotide sequences are observed in a significant number of nucleic acid samples from a population of subjects. A polymorphic site may be a nucleotide sequence of two or more nucleotides, an inserted nucleotide or nucleotide sequence, a deleted nucleotide or nucleotide sequence, or a microsatellite, for example. A polymorphic site may be two or more nucleotides in length, may be 3,4, 5, 6, 7, 8, 9, 10, 11, 12, 13, 14, 15 or more, 20 or more, 30 or more, 50 or more, 75 or more, 100 or more, 500 or more, or about 1000 nucleotides in length, where all or some of the nucleotide sequences differ within the region. A polymorphic site is often one nucleotide in length, which is referred to herein as a single nucleotide polymorphism (SNP).

Where there are two, three, or four alternative nucleotide sequences at a polymorphic site, each nucleotide sequence is referred to as a "polymorphic variant" or "nucleic acid variant." Where two polymorphic variants exist, for example, the polymorphic variant represented in a minority of samples from a population is sometimes referred to as a "minor allele" and the polymorphic variant that is more prevalently represented is sometimes referred to as a "major allele." Many organisms possess a copy of each chromosome (e.g., humans), and those subjects who possess two major alleles or two minor alleles are often referred to as being "homozygous" with respect to the polymorphism, and those subjects who possess one major allele and one minor allele are normally referred to as being "heterozygous" with respect to the polymorphism. Individuals who are homozygous with respect to one allele are sometimes predisposed to a different phenotype as compared to subjects who are heterozygous or homozygous with respect to another allele.

The term "genotype" refers to the alleles present in DNA from a subject or patient, where an allele can be defined by the particular nucleotide(s) present in a nucleic acid sequence at a particular site(s). Often a genotype is the nucleotide(s) present at a single polymorphic site known to vary in the human population.

Furthermore, a genotype or polymorphic variant may be expressed in terms of a "haplotype," which as used herein refers to two or more polymorphic variants occurring within genomic DNA in a group of subjects within a population. For example, two SNPs may exist within a gene where each SNP position includes a cytosine variation and an adenine variation. Certain subjects in a population may carry one allele (heterozygous) or two alleles (homozygous) having the gene with a cytosine at each SNP position. As the two cytosines corresponding to each SNP in the gene travel together on one or both alleles in these subjects, the subjects can be characterized as having a cytosine/cytosine haplotype with respect to the two SNPs in the gene.

As used herein, the term "phenotype" refers to a trait which can be compared between subjects, such as presence or absence of a condition, a visually observable difference in appearance between subjects, metabolic variations, physiological variations, variations in the function of biological molecules, and the like. An example of a phenotype is occurrence of breast cancer. For example, a phenotype of a homozygous CYP1B1*3/*3 variant is non-responsive to docetaxel, whereas a phenotype of a homozygous wild type or a heterozygous subject is responsive to docetaxel.

The terms "variant form of a gene," "form of a gene," or "allele" refer to one specific form of a gene in a population, the specific form differing from other forms of the same gene in the sequence of at least one, and frequently more than one, variant sites within the sequence of the gene. The sequences at these variant sites that differ between different alleles of the gene are termed "gene sequence variances" or "variances" or "variants." The term "alternative form" refers to an allele that can be distinguished from other alleles by having distinct variances at least one, and frequently more than one, variant sites within the gene sequence. Other terms known in the art to be equivalent include mutation and polymorphism, although mutation is often used to refer to an allele associated with a deleterious phenotype. In the methods utilizing variance presence or absence, reference to the presence of a variance or variances means particular variances, e.g., particular nucleotides at particular polymorphic sites, rather than just the presence of any variance in the gene.

Variances occur in the human genome at approximately one in every 500-1,000 bases within the human genome when two alleles are compared. When multiple alleles from unrelated subjects are compared the density of variant sites increases as different subjects, when compared to a reference sequence, will often have sequence variances at different sites. At most variant sites there are only two alternative nucleotides involving the substitution of one base for another or the insertion/deletion of one or more nucleotides. Within a gene there may be several variant sites. Variant forms of the gene or alternative alleles can be distinguished by the presence of alternative variances at a single variant site, or a combination of several different variances at different sites (haplotypes).

The term "haplotype" refers to a cis arrangement of two or more polymorphic nucleotides, e.g., variances, on a particular chromosome, e.g., in a particular gene. The haplotype preserves information about the phase of the polymorphic nucleotides, that is, which set of variances were inherited from one parent, and which from the other. A genotyping test does not provide information about phase. For example, an subject heterozygous at nucleotide 25 of a gene (both A and C are present) and also at nucleotide 100 (both G and T are present) could have haplotypes 25A-100G and 25C-100T, or alternatively 25A-100T and 25C-100G. Phase can also be predicted statistically based on calculations of linkage frequencies, and the most likely phase can be assessed by such methods as well.

A polymorphic variant may be detected on either or both strands of a double-stranded nucleic acid. For example, a thymine at a particular position in a sequence can be reported as an adenine from the complementary strand. Also, a polymorphic variant may be located within an intron or exon of a gene or within a portion of a regulatory region such as a promoter, a 5' untranslated region (UTR), a 3' UTR, and in DNA (e.g., genomic DNA (gDNA) and complementary DNA (cDNA)), RNA (e.g., mRNA, tRNA, and rRNA), or a polypeptide. Polymorphic variations may or may not result in detectable differences in gene expression, polypeptide structure, or polypeptide function.

The terms "disease" or "condition" are commonly recognized in the art and designate the presence of signs and/or symptoms in a subject or patient that are generally recognized as abnormal. Diseases or conditions may be diagnosed and categorized based on pathological changes. Signs may include any-objective evidence of a disease such as changes that are evident by physical examination of a patient or the results of diagnostic tests which may include, among others, laboratory tests to determine the presence of DNA sequence variances or variant forms of certain genes in a patient. Symptoms are subjective evidence of disease or a patients condition, e.g., the patients perception of an abnormal condition that differs from normal function, sensation, or appearance, which may include, without limitations, physical disabilities, morbidity, pain, and other changes from the normal condition experienced by an subject. Various diseases or conditions include, for example, those categorized in standard textbooks of medicine including, without limitation, textbooks of nutrition, allopathic, homeopathic, and osteopathic medicine. In certain aspects, the disease or condition is selected from the group consisting of the types of diseases listed in standard texts such as Harrison's Principles of Internal Medicine (14th Ed) by Anthony S. Fauci, Eugene Braunwald, Kurt J. Isselbacher, et al. (Editors), McGraw Hill, 1997, or Robbins Pathologic Basis of Disease (6th edition) by Ramzi S. Cotran, Vinay Kumar, Tucker Collins & Stanley L. Robbins, W B Saunders Co., 1998, or the Diagnostic and Statistical Manual of Mental Disorders: DSM-IV (.sub.4th edition), American Psychiatric Press, 1994, or other texts described below.

The phrase "suffering from a disease or condition" means that a subject is either presently subject to the signs and symptoms, or is more likely to develop such signs and symptoms than a normal subject in the population. Thus, for example, a subject suffering from a condition can include a developing fetus, a subject to a treatment or environmental condition which enhances the likelihood of developing the signs or symptoms of a condition, or a subject who is being given or will be given a treatment which increase the likelihood of the subject developing a particular condition. Thus, methods of the present invention which relate to treatments of patients (e.g., methods for selecting a treatment, selecting a patient for a treatment) can include primary treatments directed to a presently active disease or condition, secondary treatments which are intended to cause a biological effect relevant to a primary treatment, and prophylactic treatments intended to delay, reduce, or prevent the development of a disease or condition, as well as treatments intended to cause the development of a condition different from that which would have been likely to develop in the absence of the treatment.

In certain embodiments, the disease or condition is one which is treatable by a tubulin stabilization agent, for example, cancer, e.g., prostate, breast, and/or lung cancer.

The term "therapy" refers to a process that is intended to produce a beneficial change in the condition of a mammal, e.g., a human, often referred to as a patient. A beneficial change can, for example, include one or more of restoration of function, reduction of symptoms, limitation or retardation of progression of a disease, disorder, or condition or prevention, limitation or retardation of deterioration of a patient's condition, disease or disorder. Such therapy can involve, for example, nutritional modifications, administration of radiation, administration of a drug, behavioral modifications, and combinations of these, among others.

The terms "drug" and "therapeutic agent," as used herein refer to a chemical entity or biological product, or combination of chemical entities or biological products, administered to a subject to treat or prevent or control a disease or condition, e.g., an anti-neoplastic agent. The chemical entity or biological product is preferably, but not necessarily a low molecular weight compound, but may also be a larger compound, for example, an oligomer of nucleic acids, amino acids, or carbohydrates including without limitation proteins, oligonucleotides, ribozymes, DNAzymes, glycoproteins, lipoproteins, and modifications and combinations thereof. A biological product is preferably a monoclonal or polyclonal antibody or fragment thereof such as a variable chain fragment or single chain antibody, nanobody; cells; or an agent or product arising from recombinant technology, such as, without limitation, a recombinant protein, recombinant vaccine, or DNA construct developed for therapeutic, e.g., human therapeutic, use. The term "drug" may include, without limitation, compounds that are approved for sale as pharmaceutical products by government regulatory agencies (e.g., U.S. Food and Drug Administration (FDA), European Medicines Evaluation Agency (EMEA), and a world regulatory body governing the International Conference of Harmonization (ICH) rules and guidelines), compounds that do not require approval by government regulatory agencies, food additives or supplements including compounds commonly characterized as vitamins, natural products, and completely or incompletely characterized mixtures of chemical entities including natural compounds or purified or partially purified natural products. The term "drug" as used herein is synonymous with the terms "medicine," "pharmaceutical product," or "product." Most preferably the drug is approved by a government agency for treatment of a specific disease or condition. Included are "candidate compounds" or "candidate tubulin stabilization agents," refers to a drug, agent or compound that is under investigation, either in laboratory or human clinical testing for a specific disease, disorder, or condition.

The term "probe," as used herein, refers to a molecule which detectably distinguishes between target molecules differing in structure. Detection can be accomplished in a variety of different ways depending on the type of probe used and the type of target molecule. Thus, for example, detection may be based on discrimination of activity levels of the target molecule, but preferably is based on detection of specific binding. Examples of such specific binding include antibody binding and nucleic acid probe hybridization. Thus, for example, probes can include enzyme substrates, antibodies and antibody fragments, and nucleic acid hybridization probes. Thus, in preferred embodiments, the detection of the presence or absence of the at least one variance involves contacting a nucleic acid sequence which includes a variance site with a probe, preferably a nucleic acid probe, where the probe preferentially hybridizes with a form of the nucleic acid sequence containing a complementary base at the variance site as compared to hybridization to a form of the nucleic acid sequence having a non-complementary base at the variance site, where the hybridization is carried out under selective hybridization conditions. Such a nucleic acid hybridization probe may span two or more variance sites. Unless otherwise specified, a nucleic acid probe can include one or more nucleic acid analogs, labels or other substituents or moieties so long as the base-pairing function is retained. For example, techniques such as OLA, TAQMAN, and methods described in US Patent Application Publication No. 2004/0121371 are also useful detection methods according to the methods disclosed herein.

As used herein the term "chemical class" refers to a group of compounds that share a common chemical scaffold but which differ in respect to the substituent groups linked to the scaffold. Examples of chemical classes of drugs include, for example, phenothiazines, piperidines, benzodiazepines and aminoglycosides. Members of the phenothiazine class include, for example, compounds such as chlorpromazine hydrochloride, mesoridazine besylate, thioridazine hydrochloride, acetophenazine maleate trifluoperazine hydrochloride and others, all of which share a phenothiazine backbone. Members of the piperidine class include, for example, compounds such as meperidine, diphenoxylate and loperamide, as well as phenylpiperidines such as fentanyl, sufentanil and alfentanil, all of which share the piperidine backbone. Chemical classes and their members are recognized by those skilled in the art of medicinal chemistry. A preferred chemical class is that to which docetaxel belongs, e.g., the taxoid family. For example, see US Patent Nos: 4814470, 5438072, 5698582, and 5714512.

"Predicting responsiveness of a tumor," refers to, for example, the determination or forecasting of whether a tumor may react to a particular treatment. For example, this may be based on the genotype of a particular gene, for example, CYP1B1.

"Genotype status," as used herein refers to the particular genotype of a subject, a tissue of a subject and/or of a cell of a subject. The genotype may be of just one gene, or may be of many genes. For example, the genotype status may be of CYP1B1 and determined by detecting the presence or absence of a variation at nucleotide position 4326 or amino acid position 432. The wildtype genotype status of CYP1B1 is L at amino acid 432 and C at nucleotide 4236, and the variant CYP1B1*3 is amino acid V at 432 and nucleotide G at 4236. The genotype status may be determined, for example, by biochemical methods, e.g., array based methods, PCR based methods, and other method now known or later developed in the art.

"Anti-neoplastic agent," as used herein is an agent that will halt tumor growth, slow tumor growth, kill tumor cells, cause tumor cells to enter apoptosis, limit the blood supply to tumors and the like. Examples include, docetaxel, cisplatin, cyclophosphamind, doxorubicin, prednisone, 5-FU, trastuzumab (Herceptin TM), 3G4 (travacin equivalent) travacin, gemcitabine, estramustine, carboplatin, radiation.

"Co-administering," as used herein refers to the administration with another agent, either at the same time, in the same composition, at alternating times, in separate compositions, or combinations thereof.

"One or more additional anti-neoplastic agents," refers to the selection of additional therapeutic agents that may be co-administered with the tubulin stabilization agent are selected from cisplatin, mitomycin, capecitabine, irinotecan, topotecan, estramustine, vinorelbine, cyclophosphamide, ifosfamide, doxorubicin, epirubicin, 5-FU, trastuzumab (Herceptin TM), 3G4 (travacin equivalent) travacin, gemcitabine, estramustine, carboplatin, imatinib, gefitinib, erlotinib, cetuximab (Erbitux), bevacizumab (Avastin), thalidomide, or radiation.

As used herein, the terms "tumor" or "cancer" refer to a condition characterized by anomalous rapid proliferation of abnormal cells in one or both breasts of a subject. The abnormal cells often are referred to as "neoplastic cells," which are transformed cells that can form a solid tumor. The term "tumor" refers to an abnormal mass or population of cells (e.g., two or more cells) that result from excessive or abnormal cell division, whether malignant or benign, and pre-cancerous and cancerous cells. Malignant tumors are distinguished from benign growths or tumors in that, in addition to uncontrolled cellular proliferation, they can invade surrounding tissues and can metastasize. In breast cancer, neoplastic cells may be identified in one or both breasts only and not in another tissue or organ, in one or both breasts and one or more adjacent tissues or organs (e.g. lymph node), or in a breast and one or more non-adjacent tissues or organs to which the breast cancer cells have metastasized. As used herein, "tumor" or "cancer," refers to one or more of breast, prostate, lung, head and neck, mesothelioma, ovarian, urothelial, hepatocellular, bladder, esopheageal, or stomach.

The term "invasion" as used herein refers to the spread of cancerous cells to adjacent surrounding tissues. The term "invasion" often is used synonymously with the term "metastasis," which as used herein refers to a process in which cancer cells travel from one organ or tissue to another non-adjacent organ or tissue. Cancer cells in the breast(s) can spread to tissues and organs of a subject, and conversely, cancer cells from other organs or tissue can invade or metastasize to a breast. Cancerous cells from the breast(s) may invade or metastasize to any other organ or tissue of the body. Breast cancer cells often invade lymph node cells and/or metastasize to the liver, brain and/or bone and spread cancer in these tissues and organs. Breast cancers can spread to other organs and tissues and cause lung cancer, prostate cancer, colon cancer, ovarian cancer, cervical cancer, gastrointestinal cancer, pancreatic cancer, glioblastoma, bladder cancer, hepatoma, colorectal cancer, uterine cervical cancer, endometrial carcinoma, salivary gland carcinoma, kidney cancer, vulval cancer, thyroid cancer, hepatic carcinoma, skin cancer, melanoma, ovarian cancer, neuroblastoma, myeloma, various types of head and neck cancer, acute lymphoblastic leukemia, acute myeloid leukemia, Ewing sarcoma and peripheral neuroepithelioma, and other carcinomas, lymphomas, blastomas, sarcomas, and leukemias.

Prostate cancer, along with lung and colon cancer, are the three most common causes of death from cancer in men in the U.S., but prostate is by far the most prevalent of all human malignancies with the exception of skin cancer (Scott R. et al., J. Urol., 101:602,1969; Sakr W A et al., J. Urol., 150: 379, 1993). It is one of the top three causes of death from cancer in men in the United States (Greenlee R T et al., CA Cancer J. Clin. Vol 15, 2001) Currently, treatments available for prostate cancer require not only an early detection of the malignancy and a reliable assessment of the severity of the cancer.

As used herein, "assessing the risk of cancer in a subject," refers to, for example, the determination of,the clinical outcome based on percentages of, for example, survival given their genotype and treatment options.

As used herein, the phrase "tubulin stabilization agent," includes the class of compounds that function similarly to and are in the same chemical class as, for example, docetaxel, paclitaxel, and taxane and epothilone analogues

"Assessing the responsiveness of a subject to treatment with a tubulin stabilization agent," may be done by any clinical or biological method. For example, a reduction in tumor size, cessation of tumor growth, reduction in symptoms (e.g., pain, bleeding, amenia, tumor growth), and/or by in vitro cell based methods.

"Providing," refers to obtaining, by for example, buying or making the, e.g., polypeptide, drug, polynucleotide, probe, and the like. The material provided may be made by any known or later developed biochemical or other technique. For example, polypeptides may be obtained from cultured cells. The cultured cells, for example, may comprise an expression construct comprising a nucleic acid segment encoding the polypeptide.

Cells and/or subjects may be treated and/or contacted with one or more anti-neoplastic treatments including, surgery, chemotherapy, radiotherapy, gene therapy, immune therapy or hormonal therapy, or other therapy recommended or proscribed by self or by a health care provider.

As used herein, "treating, preventing or alleviating cancer," refers to the prophylactic or therapeutic use of the therapeutic agents described herein, e.g., tubulin stabilization agents.

"Substantially purified" when used in the context of a polypeptide or polynucleotide, or fragment or variant thereof that are at least 60% free, preferably 75% free and more preferably 90% free from other components with which they are naturally associated. An "isolated polypeptide" or "isolated polynucleotide" is, therefore, a substantially purified polypeptide or polynucleotide, respectively.

The term "subject" includes organisms which are capable of suffering from cancer or who could otherwise benefit from the administration of a compound or composition herein described, such as human and non-human animals. Preferred human animals include human patients suffering from or prone to suffering from cancer or associated state, as described herein. The term "non-human animals" includes all vertebrates, *e.g*., mammals, *e.g*., rodents, *e.g.,* mice, and non-mammals, such as non-human primates, *e.g.,* sheep, dog, cow, chickens, amphibians, reptiles, *etc.*

A method for "predicting" or "diagnosing" as used herein refers to a clinical or other assessment of the condition of a subject based on observation, testing, or circumstances.

"Determining a level of expression" or "determining a genotype," may be by any now known or hereafter developed assay or method of determining expression level, for example, immunological techniques, PCR techniques, immunoassay, quantitative immunoassay, Western blot or ELISA, quantitative RT-PCR, and/or Northern blot. The level may be ofRNA or protein. sequencing, real-time PCR, PCR, allele-specific PCR, Pyrosequencing, SNP Chip technology, or RFLP

A sample or samples may be obtained from a subject, for example, by swabbing, biopsy, lavage or phlebotomy. Samples include tissue samples, blood, sputum, bronchial washings, biopsy aspirate, or ductal lavage.

"Therapeutically effective amount," as used herein refers to an amount of an agent which is effective, upon single or multiple dose administration to the cell or subject, in prolonging the survivability of the patient with such a disorder beyond that expected in the absence of such treatment.

Compositions described herein may be administered, for example, systemically, intratumorally, intravascularally, to a resected tumor bed, orally, or by inhalation.

As used herein, the term "primer" refers to an oligonucleotide, whether occurring naturally as in a purified restriction digest or produced synthetically, which is capable of acting as a point of initiation of synthesis when placed under conditions in which synthesis of a primer extension product which is complementary to a nucleic acid strand is induced, (e.g., in the presence of nucleotides and an inducing agent such as DNA polymerase and at a suitable temperature and pH). The primer is preferably single stranded for maximum efficiency in amplification, but may alternatively be double stranded. If double stranded, the primer is first treated to separate its strands before being used to prepare extension products. The primer must be sufficiently long to prime the synthesis of extension products in the presence of the inducing agent. The exact lengths of the primers will depend on many factors, including temperature, source of primer and the use of the method.

Unless otherwise indicated, a particular nucleic acid sequence also implicitly encompasses conservatively modified variants thereof (e.g., degenerate codon substitutions) and complementary sequences, as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res., 19:5081 (1991); Ohtsuka et al., J. Boil. Chem. 260:2605-2608 (1985); Rossolini et al., Mol. Cell Probes, 8:91-98 (1994)). The term nucleic acid is used interchangeably with gene, cDNA, mRNA, oligonucleotide, and polynucleotide.

The terms "polypeptide," "peptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer.

As used herein, the term "polymerase chain reaction" (PCR) refers to the methods of U.S. Pat. Nos. 4,683,195, 4,683,202, and 4,965,188, directed to methods for increasing the concentration of a segment of a target sequence in a mixture of genomic DNA without cloning or purification. As used herein, the terms "PCR product" and "amplification product" refer to the resultant mixture of compounds after two or more cycles of the PCR steps of denaturation, annealing and extension are complete. These terms encompass the case where there has been amplification of one or more segments of one or more target sequences.

As used herein, the term "recombinant DNA molecule" as used herein refers to a DNA molecule, which is comprised of segments of DNA joined together by means of molecular biological techniques.

As used herein, a nucleic acid sequence, even if internal to a larger oligonucleotide, also may be said to have 5' and 3' ends. In either a linear or circular DNA molecule, discrete elements are referred to as being "upstream" or 5' of the "downstream" or 3' elements. This terminology reflects the fact that transcription proceeds in a 5' to 3' fashion along the DNA strand. The promoter and enhancer elements which direct transcription of a linked gene are generally located 5' or upstream of the coding region. However, enhancer elements can exert their effect even when located 3' of the promoter element and the coding region. Transcription termination and polyadenylation signals are located 3' or downstream of the coding region.

As used herein, an oligonucleotide having a nucleotide sequence encoding a gene refers to a DNA sequence comprising the coding region of a gene or in other words the DNA sequence, which encodes a gene product. The coding region may be present in either a cDNA or genomic DNA form. Suitable control elements such as enhancers/promoters, splice junctions, polyadenylation signals, etc., may be placed in close proximity to the coding region of the gene if needed to permit proper initiation of transcription and/or correct processing of the primary RNA transcript. Alternatively, the coding region utilized in the vectors may contain endogenous enhancers/promoters, splice junctions, intervening sequences, polyadenylation signals, etc., or a combination of both endogenous and exogenous control elements.

The terms "identical" or percent "identity," in the context of two or more nucleic acids or polypeptide sequences, refer to two or more sequences or subsequences that are the same or have a specified percentage of amino acid residues or nucleotides that are the same (e.g., 60% identity, optionally 65%, 70%, 75%, 80%, 85%, 90%, or 95% identity over a specified region), when compared and aligned for maximum correspondence over a comparison window, or designated region as measured using one of the following sequence comparison algorithms or by manual alignment and visual inspection. Such sequences are then said to be "substantially identical." This definition also refers to the compliment of a test sequence. Optionally, the identity exists over a region that is at least about 50 amino acids or nucleotides in length, or more preferably over a region that is 75-100 amino acids or nucleotides in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters.

A "comparison window," as used herein, includes reference to a segment of any one of the number of contiguous positions selected from the group consisting of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are well-known in the art. Optimal alignment of sequences for comparison can be conducted, e.g., by the local homology algorithm of Smith & Waterman, Adv. Appl. Math., 2:482 (1981), by the homology alignment algorithm of Needleman & Wunsch, J. Mol Biol., 48:443 (1970), by the search for similarity method of Pearson & Lipman, Proc. Natl. Acad. Sci. U.S.A., 85:2444 (1988), by computerized implementations of these algorithms (GAP, BESTFIT, FASTA, and TFASTA in the Wisconsin Genetics Software Package, Genetics Computer Group, 575 Science Dr., Madison, Wis.), or by manual alignment and visual inspection (see, e.g., Current Protocols in Molecular Biology (Ausubel et al., eds. 1995 supplement)).

As used herein, the term "antibody" refers to any molecule which has specific immunoreactivity activity, whether or not it is coupled with another compound such as a targeting agent, carrier, label, toxin, or drug. Although an antibody usually comprises two light and two heavy chains aggregated in a "Y" configuration with or without covalent linkage between them, the term is also meant to include any reactive fragment or fragments of the usual composition, such as Fab molecules, Fab proteins or single chain polypeptides having binding affinity for an antigen. Fab refers to antigen binding fragments. As used herein, the term "Fab molecules" refers to regions of antibody molecules which include the variable portions of the heavy chain and/or light chain and which exhibit binding activity. "Fab protein" includes aggregates of one heavy and one light chain (commonly known as Fab), as well as tetramers which correspond to the two branch segments of the antibody Y (commonly known as F(ab)₂), whether any of the above are covalently or non-covalently aggregated so long as the aggregation is capable of selectively reacting with a particular antigen or antigen family.

The term "antibodies" is used herein in a broad sense and includes both polyclonal and monoclonal antibodies. In addition to intact immunoglobulin molecules, also included in the term "antibodies" are fragments or polymers of those immunoglobulin molecules, and human or humanized versions of immunoglobulin molecules or fragments thereof, as long as they are chosen for their ability to interact with the proteins disclosed herein. The antibodies can be tested for their desired activity using the in vitro assays described herein, or by analogous methods, after which their in vivo therapeutic and/or prophylactic activities are tested according to known clinical testing methods.

The antibodies are raised against the different alleles of CYP1B1, e.g., CYP1B1*3/*3 and wild-type CYP1B1. The antibody can be a polyclonal, monoclonal, recombinant, e.g., a chimeric or humanized, fully human, non-human, e.g., murine, single chain antibody, or fully synthetic. Chimeric, humanized, but most preferably, completely human antibodies are desirable for applications which include repeated administration, e.g., therapeutic treatment of human patients, and some diagnostic applications. In a related embodiment, the antibody can be coupled to a toxin.

### Methods of Predicting Responsiveness, Selecting Subjects, and Assessing Risks of Treatments

Many drugs or other treatments are known to have highly variable safety and efficacy in different subjects. A consequence of such variability is that a given drug or other treatment may be effective in one subject, and ineffective or not well-tolerated in another subject. Thus, administration of such a drug to a subject in whom the drug would be ineffective would result in wasted cost and time during which the patient's condition may significantly worsen. Also, administration of a drug to an subject in whom the drug would not be tolerated could result in a direct worsening of the patient's condition and could even result in the patient's death.

For some drugs, over 90% of the measurable intersubject variation in selected pharmacokinetic parameters has been shown to be heritable. For a limited number of drugs, DNA sequence variances have been identified in specific genes that are involved in drug action or metabolism, and these variances have been shown to account for the variable efficacy or safety of the drugs in different subjects. As the sequence of the human genome is completed, and as additional human gene sequence variances are identified, the power of genetic methods for predicting drug response will further increase. This application concerns methods for identifying and exploiting gene sequence variances that account for interpatient variation in drug response, particularly interpatient variation attributable to pharmacokinetic factors and interpatient variation in drug tolerability or toxicity.

The efficacy of a drug is a function of both pharmacodyniamic effects and pharmacokinetic effects, or bioavailability. In the present invention, interpatient variability in drug safety, tolerability and efficacy are discussed in terms of the genetic determinants of interpatient variation in absorption, distribution, metabolism, and excretion, e.g., pharmacokinetic parameters.

Adverse drug reactions are a principal cause of the low success rate of drug development programs (less than one in four compounds that enters human clinical testing is ultimately approved for use by the US Food and Drug Administration (FDA)). Adverse drug reactions can be categorized as 1) mechanism based reactions and 2) idiosyncratic, "unpredictable" effects apparently unrelated to the primary pharmacologic action of the compound. Although some side effects appear shortly after administration, in some instances side effects appear only after a latent period.

Similarly, many compounds are not approved due to unimpressive efficacy. The identification of genetic determinants of pharmacokinetic variation may lead to identification of a genetically defined population in whom a significant response is occurring. Approval of a compound for this population, defined by a genetic diagnostic test, may be the only means of getting regulatory approval for a drug. As healthcare becomes increasingly costly, the ability to allocate healthcare resources effectively becomes increasingly urgent. The use of genetic tests to develop and rationally administer medicines represents a powerful tool for accomplishing more cost effective medical care.

Thus, in one aspect, the invention provides a method for selecting a treatment for a patient suffering from a disease or condition by determining whether or not a gene or genes in cells of the patient (in some cases including both normal and disease cells, such as cancer cells) contain at least one sequence variance which is indicative of the effectiveness of the treatment of the disease or condition. The methods disclosed herein may be used with other genotyping or tumor marker methods if necessary. Preferably the at least one variance includes a plurality of variances which may provide a haplotype or haplotypes. Preferably the joint presence of the plurality of variances is indicative of the potential effectiveness or safety of the treatment in a patient having such plurality of variances. The plurality of variances may each be indicative of the potential effectiveness of the treatment, and the effects of the subject variances may be independent or additive, or the plurality of variances may be indicative of the potential effectiveness if at least 2, 3, 4, or more appear jointly. The plurality of variances may also be combinations of these relationships. The plurality of variances may include variances from one, two, three or more gene loci.

In another aspect, methods of predicting responsiveness of a tumor to therapeutic treatment comprise determining the genotype status of CYP1B1, and correlating the genotype to the treatment. The determining may comprise methods including, for example, array based methods, PCR based methods, immunological methods (antibodies, western blots, RIAs, etc), sequencing methods (direct and indirect sequencing of oligonucleotides or nucleic acids and peptides or proteins or Pyrosequencing), expression level of CYP1B1 alleles, enzyme kinetics of CYP1B1 (enzyme kinetics of CYP1B1 toward several substrates are describe, for example, in references 8-11), PCR methods (real-time PCR, allele-specific PCR, reverse-transcriptase PCR, PCR), SNP Chip technology, RFLP and/or other assays described herein. The genotype status, refers to, for example, the genotype of one or both alleles of a humans CYP1B1 gene. The genotype status of CYP1B1 may comprise determining the identity of the nucleotide position 4326 of CYP1B1 and/or determining the identity of the amino acid position 432. The assay may be informative if only one allele is determined. For example, if only one allele is determined and it is wild-type, the assay is informative because both a heterozygous subject and a homozygous wild-type subj ect will be correlated with a positive response to the tubulin stabilization agent. If only one allele is determined to be CYP1B1*3, it may be unclear whether or not to treat the subject with a tubulin stabilization agent because the subject may be a heterozygous or a homozygous variant. A wild-type or heterozygous status correlates with responsiveness of a tumor to therapeutic treatment and a CYP1B1*3/*3 status correlates with unresponsiveness of a tumor to therapeutic treatment. The wildtype is L at 432 and C at 4236 and the CYP1B1*3 is V at 432 and G at 4236.

"Correlating," "correlation," "correlates," as used herein refer to the establishment of mutual or reciprocal relationship between genotype status and therapeutic efficacy of certain treatments as described herein. That is, correlating refers to relating the genotype status to risk, treatment.

As used herein, "homozygous variant CYP1B1 genotype status," refers to the CYP1B1*3/*3 variant being found on both alleles of CYP1B1. "CYP1B1*3" or "CYP1B1*3/*3" is sometimes used as a short-hand for "homozygous variant CYP1B1 genotype status."

Methods described herein may further comprise administering a therapeutic amount of an anti-neoplastic agent to the subject. For example, a tubulin stabilization agent, e.g., docetaxel, paclitaxel, or other taxane derivative. Methods may further comprise co-administering one or more additional anti-neoplastic agents to the subject. One or more additional anti-neoplastic agents may be selected from, for example, cisplatin, cyclophosphamind, doxorubicin, prednisone, 5-FU, trastuzumab (Herceptin TM), 3G4 (travacin equivalent) travacin, gemcitabine, estramustine, carboplatin, and/or radiation. The treatment may be individualized to the subject an other anti-neoplastic agents may be co-administered.

In another aspect, methods of selecting a subject that will respond to docetaxel treatment are provided. The methods comprise detecting the presence or absence of a variation at nucleotide position 4326 or amino acid position 432 of CYP1B1 and correlating the absence of a variation or heterozygous variation with an indication that the subject will respond to docetaxel treatment. The methods may further comprise correlating the presence of a variation with an indication that the subject will not respond to docetaxel treatment. Preferably, both alleles of the CYP1B1 gene are determined. The assay, however, maybe informative if only one allele is determined. For example, if only one allele is determined and it is wild-type, the assay is informative because both heterozygous subjects and homozygous wild-type subjects are correlated with a positive response to the tubulin stabilization agent. If only one allele is determined to be CYP1B1*3/*3, it may be unclear whether or not to treat the subject with a tubulin stabilization agent because the subject may be a heterozygous or a homozygous variant. That is, a wild-type or heterozygous variation correlates with responsiveness of a tumor to therapeutic treatment and a CYP1B1*3/*3 status correlates with unresponsiveness of a tumor to therapeutic treatment. The wildtype is L at amino acid 432 and C at nucleotide 4236 and the CYP1B1*3 is V at amino acid 432 and G at nucleotide 4236. Selection of subjects maybe from, for example, individuals suffering from, being diagnosed as suffering from or suspected of suffering from cancer. For example, breast, prostate, lung, head and neck, mesothelioma, ovarian, urothelial, hepatocellular, bladder, esopheageal, or stomach cancer. Subjects may also be selected from individuals that are matched controls, e.g., for clinical trials of tubulin stabilization agents.

Methods of assessing the risk of cancer in a subject are also presented and comprise determining the genotype status of CYP1B1, and correlating the genotype status to cancer risk. A subject has increased risk if they are determined to be homozygous for CYP1B1*3/*3 because they are not likely to respond to treatment with docetaxel or other tubulin stabilization agents.

Tubulin stabilization agents may be selected from one or more of docetaxel, paclitaxel, docetaxel, or other taxane derivative.

In some cases, the selection of a method of treatment or assessment, e.g., a therapeutic regimen, may incorporate selection of one or more from a plurality of medical therapies. Thus, the selection may be the selection of a method or methods which is/are more effective or less effective than certain other therapeutic regimens (with either having varying safety parameters). Likewise or in combination with the preceding selection, the selection may be the selection of a method or methods, which is safer than certain other methods of treatment in the patient.

The selection may involve either positive selection or negative selection or both, meaning that the selection can involve a choice that a particular method would be an appropriate method to use and/or a choice that a particular method would be an inappropriate method to use. Thus, in certain embodiments, the presence of the at least one variance is indicative that the treatment will be effective or otherwise beneficial (or more likely to be beneficial) in the patient. Stating that the treatment will be effective means that the probability of beneficial therapeutic effect is greater than in a subject not having the appropriate presence or absence of particular variances. In other embodiments, the presence of the at least one variance is indicative that the treatment will be ineffective or contra-indicated for the patient. For example, a treatment may be contra-indicated if the treatment results, or is more likely to result, in undesirable side effects, an excessive level of undesirable side effects, and/or no beneficial results. A determination of what constitutes excessive side-effects will vary, for example, depending on the disease or condition being treated, the availability of alternatives, the expected or experienced efficacy of the treatment, and the tolerance of the patient. As for an effective treatment, this means that it is more likely that desired effect will result from the treatment administration in a patient with a particular variance or variances than in a patient who has a different variance or variances. Also in preferred embodiments, the presence of the at least one variance is indicative that the treatment is both effective and unlikely to result in undesirable effects or outcomes, or vice versa (is likely to have undesirable side effects but unlikely to produce desired therapeutic effects).

In reference to response to a treatment, the term "tolerance" refers to the ability of a patient to accept a treatment, based, e.g., on deleterious effects and/or effects on lifestyle. Frequently, the term principally concerns the patients perceived magnitude of deleterious effects such as nausea, weakness, dizziness, and diarrhea, among others. Such experienced effects can, for example, be due to general or cell- specific toxicity, activity on non-target cells, cross-reactivity on non-target cellular constituents (non-mechanism based), and/or side effects of activity on the target cellular substituents (mechanism based), or the cause of toxicity may not be understood. In any of these circumstances one may identify an association between the undesirable effects and variances in specific genes.

Also in other embodiments, the method of selecting a treatment includes eliminating a treatment, where the presence or absence of the at least one variance is indicative that the treatment will be ineffective or contra-indicated, e.g., would not reduce tumor size, would not stop tumor growth, and/or metastasis. In other preferred embodiments, in cases in which undesirable side-effects may occur or are expected to occur from a particular therapeutic treatment, the selection of a method of treatment can include identifying both a first and second treatment, where the first treatment is effective to treat the disease or condition, and the second treatment reduces a deleterious effect of the first treatment.

The phrase "eliminating a treatment" refers to removing a possible treatment from consideration, e.g., for use with a particular patient based on the presence or absence of a particular variance(s) in one or more genes in cells of that patient, or to stopping the administration of a treatment which was in the course of administration.

Usually, the treatment will involve the administration of a compound preferentially active or safe in patients with a form or forms of a gene, where the gene is identified herein. The administration may involve a combination of compounds. Thus, in preferred embodiments, the method involves identifying such an active compound or combination of compounds, where the compound is less active or is less safe or both when administered to a patient having a different form of the gene.

Also in some embodiments, the method of selecting a treatment involves selecting a method of administration of a compound, combination of compounds, or pharmaceutical composition, for example, selecting a suitable dosage level and/or frequency of administration, and/or mode of administration of a compound. The method of administration can be selected to provide better, preferably maximum therapeutic benefit. In this context, "maximum" refers to an approximate local maximum based on the parameters being considered, not an absolute maximum.

Also in this context, a "suitable dosage level" refers to a dosage level which provides a therapeutically reasonable balance between pharmacological effectiveness and deleterious effects. Often this dosage level is related to the peak or average serum levels resulting from administration of a drug at the particular dosage level.

A particular gene or genes can be relevant to the treatment of more than one disease or conditions for example, the gene or genes can have a role in the initiation, development, course, treatment, treatment outcomes, or health-related quality of life outcomes of a number of different diseases, disorders, or conditions. Thus, in preferred embodiments, the disease or condition or treatment of the disease or condition is any which involves

As is generally understood, administration of a particular treatment, e.g., administration of a therapeutic compound or combination of compounds, is chosen depending on the disease or condition which is to be treated. Thus, in certain preferred embodiments, the disease or condition is one for which administration of a treatment is expected to provide a therapeutic benefit.

As used herein, the terms "effective" and "effectiveness" includes both pharmacological effectiveness and physiological safety. Pharmacological effectiveness refers to the ability of the treatment to result in a desired biological effect in the patient. Physiological safety refers to the level of toxicity, or other adverse physiological effects at the cellular, organ and/or organism level (often referred to as side-effects) resulting from administration of the treatment. On the other hand, the term "ineffective" indicates that a treatment does not provide sufficient pharmacological effect to be therapeutically useful, even in the absence of deleterious effects, at least in the unstratified population. (Such a treatment may be ineffective in a subgroup that can be identified by the presence of one or more sequence variances or alleles.) "Less effective" means that the treatment results in a therapeutically significant lower level of pharmacological effectiveness and/or a therapeutically greater level of adverse physiological effects, e.g., greater liver toxicity.

Thus, in connection with the administration of a drug, a drug which is "effective against" a disease or condition indicates that administration in a clinically appropriate manner results in a beneficial effect for at least a statistically significant fraction of patients, such as a improvement of symptoms, a cure, a reduction in disease load, reduction in tumor mass or cell numbers, extension of life, improvement in quality of life, or other effect generally recognized as positive by medical doctors familiar with treating the particular type of disease or condition.

Effectiveness is measured in a particular population. In conventional drug development the population is generally every subject who meets the enrollment criteria (e.g., has the particular form of the disease or condition being treated).

The term "deleterious effects" refers to physical effects in a patient caused by administration of a treatment which are regarded as medically undesirable. Thus, for example, deleterious effects can include a wide spectrum of toxic effects injurious to health such as death of normally functioning cells when only death of diseased cells is desired, nausea, fever, inability to retain food, dehydration, damage to critical organs such as arrvhias, renal tubular necrosis, fatty liver, or pulmonary fibrosis leading to coronary, renal, hepatic, or pulmonary insufficiency among many others. In this regard, the term "adverse reactions" refers to those manifestations of clinical symptomology of pathological disorder or dysfunction is induced by administration or a drug, agent, or candidate therapeutic intervention. In this regard, the term "contraindicated" means that a treatment results in deleterious effects such that a prudent medical doctor treating such a patient would regard the treatment as unsuitable for administration. Major factors in such a determination can include, for example, availability and relative advantages of alternative treatments, consequences of non-treatment, and permanency of deleterious effects of the treatment.

In one embodiment, the correlation of patient responses to therapy according to patient genotype is carried out in a clinical trial, e.g., as described herein according to any of the variations described. Detailed description of methods for associating variances with clinical outcomes using clinical trials are provided below. Further, in preferred embodiments the correlation of pharmacological effect (positive or negative) to treatment response according to genotype or haplotype in such a clinical trial is part of a regulatory submission to a government agency leading to approval of the drug. Most preferably the compound or compounds would not be approvable in the absence of the genetic information allowing identification of an optimal responder population.

As indicated above, in aspects of this invention involving selection of a patient for a treatment, selection of a method or mode of administration of a treatment, and selection of a patient for a treatment or a method of treatment, the selection may be positive selection or negative selection. Thus, the methods can include eliminating a treatment for a patient, eliminating a method or mode of administration of a treatment to a patient, or elimination of a patient for a treatment or method of treatment.

The term "differential" or "differentially" generally refers to a statistically significant different level in the specified property or effect. Preferably, the difference is also functionally significant. Thus, "differential binding or hybridization" is a sufficient difference in binding or hybridization to allow discrimination using an appropriate detection technique. Likewise, "differential effect" or "differentially active" in connection with a therapeutic treatment or drug refers to a difference in the level of the effect or activity which is distinguishable using relevant parameters and techniques for measuring the effect or activity being considered. Preferably the difference in effect or activity is also sufficient to be clinically significant, such that a corresponding difference in the course of treatment or treatment outcome would be expected, at least on a statistical basis.

Also provided, are methods for determining the therapeutic capacity of a tubulin stabilization agent to reduce tissue degeneration in a subject, comprising determining pre-treatment levels of tumor status in a subject; administering a therapeutically effective amount of a tubulin stabilization agent to the subject; and determining a post-treatment level of tumor in the subject.

In certain embodiments, a modulation of tumor status indicates that the tubulin stabilization agent is efficacious. In other embodiments, the pre-treatment and post-treatment levels of tumor status are determined in a diseased tissue, e.g., a tumor mass, blood, prostate, fetus, lung, heart, liver, breast, vasculature and/or nervous tissue.

In other aspects, methods for determining the therapeutic capacity of a candidate tubulin stabilization agent for treating cancer are provided and comprise providing a population of tumor cells with a known CYP1B1 genotype; contacting the cells with a candidate composition, and determining effect of the candidate composition on cell proliferation, wherein a decrease in cell proliferation indicates that the candidate composition may be efficacious. The method may further comprise correlating the effect with the genotype. It is possible that the efficacy of certain compounds tested will have a correlation to the CYP1B1 genotype.

The screening methods comprise providing a population of tumor cells with known genotype, and the methods may further comprise obtaining a tumor sample from a subject. The tumor cells may also be primary or established cell tumor cell lines. The method may further comprise determining the genotype of the tumor cells by the methods described herein.

### Methods

Single nucleotide polymorphism (SNP) analysis may be done, for example, by parallel screening of SNPs on micro-arrays. Differential hybridization with allele-specific oligonucleotide (ASO) probes is most commonly used in the microarray format (Pastinen et al., Genome Research 2000). The requirement for sensitivity (e.g., low detection limits) has been greatly alleviated by the development of the polymerase chain reaction (PCR) and other amplification technologies which allow researchers to amplify exponentially a specific nucleic acid sequence before analysis (for a review, see Abramson et al., Current Opinion in Biotechnology, 4:41-47 (1993)). Multiplex PCR amplification of SNP loci with subsequent hybridization to oligonucleotide arrays has been shown to be an accurate and reliable method of simultaneously genotyping at least hundreds of SNPs; see Wang et al., Science, 280:1077 (1998); see also Schafer et al., Nature Biotechnology 16:33-39 (1998).

New experimental techniques for mismatch detection with standard probes, as defined in greater detail below, include, for example, OLA, RCA, Invader^{™}, single base extension (SBE) methods, allelic PCR, and competitive probe analysis. In SBE assays, a polynucleotide probe is attached to a support and hybridized to target DNA. See also US Patent Application Publication No. 2004/0121371.

Generally, for SBE assays, probe sets are designed such that the nucleotide at the 3' end of the probe is either matched or mismatched with the queried base in the target. If the base matches and hybridizes, the DNA polymerase will extend the probe by one base in the presence of four labeled-terminator nucleotides. Alternately, if the 3' base is mismatched, the DNA polymerase does not extend the probe. Thus, the identity of the SNP or queried base in the target is determined by the probe set that is extended by the DNA polymerase.

Some probes form internal stem-loop structures resulting in target-independent self-extension of the probe thus giving a false positive signal that interferes with determination of the SNP base. The present invention aims to overcome such problems.

The polymerase chain reaction (PCR) is a widely known method for amplifying nucleic acids. Of the PCR techniques, RT-PCR (Reverse Transcription-PCR), competitive RT-PCR and the like are used for detecting and quantifying a trace amount of mRNA, and show their effectiveness.

In recent years, a real-time quantitative detection technique using PCR has been established (TaqMan PCR, Genome Res., 6 (10), 986 (1996), ABI PRISM.TM. Sequence Detection System, Applied Biosystems). This technique measures the amount of nucleic acids using a particular fluorescent-labeled probe (TaqMan probe). More specifically, this technique utilizes the following principles: For example, a fluorescent-labeled probe having a reporter dye at the 5' end and a quencher dye at the 3' end is annealed to the target DNA, and the DNA is subjected to normal PCR. As the extension reaction proceeds, the probe is hydrolyzed from the 5' end by the 5'-3' exonuclease activity possessed by DNA polymerase. As a result, the reporter dye at the 5' end is separated from the quencher dye at the 3' end, thereby eliminating the FRET (Fluorescence Resonance Energy Transfer, the reduction in fluorescence intensity owing to the decrease in the energy level of the reporter dye caused by the resonance of the two fluorescent dyes) effect produced by the spatial proximity between the two dyes, and increasing the fluorescence intensity of the reporter dye that has been controlled by the quencher dye. The target nucleic acid can be selectively quantified and detected in real-time by measuring the increase of the fluorescence intensity.

This technique is advantageous in that it can test various samples simultaneously in a short time, since, unlike the detection and quantification technique using conventional PCR it does not involve complicated steps, such as agarose gel electrophoresis of the amplified product after PCR and analysis of the electrophoresis pattern.

Generally, when conducting clinical tests in a clinical test center or the like, it is necessary to inspect an extremely large number of samples within a limited time. Therefore, there is considerable demand for the development of efficient test techniques. The real-time quantitative detection technique is a promising candidate to meet this demand.

The present inventors turned their attention to the real-time quantitative detection technique using PCR, and conceived that, if the detection technique can be utilized for detecting human P450 molecular species, the molecular species can be subjectively detected and quantified using the same apparatus under the same PCR conditions.

Determining the presence of a particular variance or plurality of variances in a particular gene in a patient can be performed in a variety of ways. In preferred embodiments, the detection of the presence or absence of at least one variance involves amplifying a segment of nucleic acid including at least one of the at least one variances. Preferably a segment of nucleic acid to be amplified is 500 nucleotides or less in length, more preferably 200 nucleotides or less, and most preferably 45 nucleotides or less. Also, preferably the amplified segment or segments includes a plurality of variances, or a plurality of segments of a gene or of a plurality of genes.

In another aspect determining the presence of a set of variances in a specific gene related to treatment of pharmnacokinetic parameters associated efficacy or safety, e.g. drug-induced disease, disorder, dysfunction, or other toxicity-related gene or genes listed in CYP1B1 may entail a haplotyping test that requires allele specific amplification of a large DNA segment of no greater than 25,000 nucleotides, preferably no greater than 10,000 nucleotides and most preferably no greater than 5,000 nucleotides. Alternatively one allele may be enriched by methods other than amplification prior to determining genotypes at specific variant positions on the enriched allele as a way of determining haplotypes. Preferably the determination of the presence or absence of a haplotype involves determining the sequence of the variant site or sites by methods such as chain terminating DNA sequencing or minisequencing, or by oligonucleotide hybridization or by mass spectrometry.

In another aspect, a method for determining a genotype of an subject in relation to one or more variances in one or more of the genes identified in above aspects by using mass spectrometric determination of a nucleic acid sequence which is a portion of a gene identified for other aspects described or a complementary sequence. Such mass spectrometric methods are known to those skilled in the art. In preferred embodiments, the method involves determining the presence or absence of a variance in a gene; determining the nucleotide sequence of the nucleic acid sequence; the nucleotide sequence is 100 nucleotides or less in length, preferably 50 or less, more preferably 30 or less, and still more preferably 20 nucleotides or less. In general, such a nucleotide sequence includes at least one variance site, preferably a variance site which is informative with respect to the expected response of a patient to a treatment as described for above aspects.

In preferred embodiments, the detection of the presence or absence of the at least one variance involves contacting a nucleic acid sequence corresponding to one of the genes identified above or a product of such a gene with a probe. The probe is able to distinguish a particular form of the gene or gene product or the presence or a particular variance or variances, e.g., by differential binding or hybridization. Thus, exemplary probes include nucleic acid hybridization probes, peptide nucleic acid probes, nucleotide-containing probes which also contain at least one nucleotide analog, and antibodies, e.g., monoclonal antibodies, and other probes as discussed herein. Those skilled in the art are familiar with the preparation of probes with particular specificities. Those skilled in the art will recognize that a variety of variables can be adjusted to optimize the discrimination between two variant forms of a gene, including changes in salt concentration, temperature, pH and addition of various compounds that affect the differential affinity of GC vs. AT base pairs, such as tetramethyl ammonium chloride. (See Current Protocols in Molecular Biology by F. M. Ausubel, R. Brent, R. E. Kingston, D. D. Moore, J. D. Seidman, K. Struhl, and V. B. Chanda (editors, John Wiley & Sons.)

In other preferred embodiments, determining the presence or absence of the at least one variance involves sequencing at least one nucleic acid sample. The sequencing involves sequencing of a portion or portions of a gene and/or portions of a plurality of genes which includes at least one variance site, and may include a plurality of such sites. Preferably, the portion is 500 nucleotides or less in length, more preferably 200 nucleotides or less, and most preferably 45 nucleotides or less in length. Such sequencing can be carried out by various methods recognized by those skilled in the art, including use of dideoxy termination methods (e.g., using dye-labeled dideoxy nucleotides) and the use of mass spectrometric methods. In addition, mass spectrometric methods may be used to determine the nucleotide present at a variance site. In preferred embodiments in which a plurality of variances is determined, the plurality of variances can constitute a haplotype or collection of haplotypes. Preferably the methods for determining genotypes or haplotypes are designed to be sensitive to all the common genotypes or haplotypes present in the population being studied (for example, a clinical trial population).

The process of genotyping involves using diagnostic tests for specific variances that have already been identified. It will be apparent that such diagnostic tests can only be performed after variances and variant forms of the gene have been identified. Identification of new variances can be accomplished by a variety of methods, alone or in combination, including, for example, DNA sequencing, SSCP, heteroduplex analysis, denaturing gradient gel electrophoresis (DGGE), heteroduplex cleavage (either enzymatic as with T4 Endonuclease 7, or chemical as with osmium tetroxide and hydroxylamine), computational methods (described in "VARIANCE SCANNING METHOD FOR IDENTIFYING GENE SEQUENCE VARIANCES" filed Oct. 14,1999, Ser. No. 09/419,705, and other methods described herein as well as others known to those skilled in the art. (See, for example: Cotton, R. G. H., Slowly but surely towards better scanning for mutations, Trends in Genetics 13(2): 43-6, 1997 or Current Protocols in Human Genetics by N. C. Dracoli, J. L. Haines, B. R. Korf, D. T. Moir, C. C. Morton, C. E. Seidman, D. R. Smith, and A. Boyle (editors), John Wiley & Sons.)

In the context of this invention, the term "analyzing a sequence" refers to determining at least some sequence information about the sequence, , e.g., determining the nucleotides present at a particular site or sites in the sequence, particularly sites that are known to vary in a population, or determining the base sequence of all of a portion of the particular sequence.

Also usefully provided herein are probes which specifically recognize a nucleic acid sequence corresponding to a variance or variances in a gene as identified in aspects above or a product expressed from the gene, and are able to distinguish a variant form of the sequence or gene or gene product from one or more other variant forms of that sequence, gene, or gene product under selective conditions. Such genes, include, for example CYP1B1, GenBank accession nos.: DQ016495; C009229; NM_012940; BC012049; AY393998. Those skilled in the art recognize and understand the identification or determination of selective conditions for particular probes or types of probes. An exemplary type of probe is a nucleic acid hybridization probe, which will selectively bind under selective binding conditions to a nucleic acid sequence or a gene product corresponding to one of the genes identified for aspects above. Another type of probe is a peptide or protein, e.g., an antibody or antibody fragment which specifically or preferentially binds to a polypeptide expressed from a particular form of a gene as characterized by the presence or absence of at least one variance. Thus, another aspect concerns such probes. In the context of this disclosure, a "probe" is a molecule, commonly a nucleic acid, though also potentially a protein, carbohydrate, polymer, or small molecule, that is capable of binding to one variance or variant form of the gene to a greater extent than to a form of the gene having a different base at one or more variance sites, such that the presence of the variance or variant form of the gene can be determined. Preferably the probe distinguishes at least one variance identified in Examples, tables or lists below or in Tables 1 or 3 of Stanton & Adams application Ser. 09/300,747.

In one embodiment, the probe is a nucleic acid probe 6, 7, 8, 9, 10, 11, 12, 13, 14, or 15, preferably at least 17 nucleotides in length, more preferably at least 20 or 22 or 25, preferably 500 or fewer nucleotides in length, more preferably 200 or 100 or fewer, still more preferably 50 or fewer, and most preferably 30 or fewer. In preferred embodiments, the probe has a length in a range from any one of the above lengths to any other of the above lengths (including endpoints). The probe specifically hybridizes under selective hybridization conditions to a nucleic acid sequence corresponding to a portion of one of the genes identified in connection with above aspects. The nucleic acid sequence includes at least one and preferably two or more variance sites. Also in preferred embodiments, the probe has a detectable label, preferably a fluorescent label. A variety of other detectable labels are known to those skilled in the art. Such a nucleic acid probe can also include one or more nucleic acid analogs.

In connection with nucleic acid probe hybridization, the term "specifically hybridizes" indicates that the probe hybridizes to a sufficiently greater degree to the target sequence than to a sequence having a mismatched base at least one variance site to allow distinguishing such hybridization. The term "specifically hybridizes," thus refers to the probe hybridizing to the target sequence, and not to non-target sequences, at a level which allows ready identification of probe/target sequence hybridization under selective hybridization conditions. Thus, "selective hybridization conditions" refer to conditions which allow such differential binding. Similarly, the terms "specifically binds" and "selective binding conditions" refer to such differential binding of any type of probe, e.g., antibody probes, and to the conditions which allow such differential binding. Typically hybridization reactions to determine the status of variant sites in patient samples are carried out with two different probes, one specific for each of the (usually two) possible variant nucleotides. The complementary information derived from the two separate hybridization reactions is useful in corroborating the results. Likewise, provided herein areisolated, purified or enriched nucleic acid sequences of 15 to 500 nucleotides in length, preferably 15 to 100 nucleotides in length, more preferably 15 to 50 nucleotides in length, and most preferably 15 to 30 nucleotides in length, which has a sequence which corresponds to a portion of one of the genes identified for aspects above. Preferably the lower limit for the preceding ranges is 17,20,22, or 25 nucleotides in length. In other embodiments, the nucleic acid sequence is 30 to 300 nucleotides in length, or 45 to 200 nucleotides in length, or 45 to 100 nucleotides in length. The nucleic acid sequence includes at least one variance site. Such sequences can, for example, be amplification products of a sequence which spans or includes a variance site in a gene identified herein. Likewise, such a sequence can be a primer, or amplification oligonucleotide which is able to bind to or extend through a variance site in such a gene. Yet another example is a nucleic acid hybridization probe comprised of such a sequence. In such probes, primers, and amplification products, the nucleotide sequence can contain a sequence or site corresponding to a variance site or sites, for example, a variance site identified herein. Preferably the presence or absence of a particular variant form in the heterozygous or homozygous state is indicative of the effectiveness of a method of treatment in a patient.

Likewise, the disclosure provides a set of primers or amplification oligonucleutides (e.g., 2, 3, 4, 6, 8, 10 or even more) adapted for binding to or extending through at least one gene identified herein.

In reference to nucleic acid sequences which "correspond" to a gene, the term "correspond" refers to a nucleotide sequence relationship, such that the nucleotide sequence has a nucleotide sequence which is the same as the reference gene or an indicated portion thereof, or has a nucleotide sequence which is exactly complementary in normal Watson-Crick base pairing, or is an RNA equivalent of such a sequence, e.g., an mRNA, or is a cDNA derived from an mRNA of the gene.

In the genetic analysis that associated cancer with the polymorphic variants described herein, samples from subjects having cancer and subjects not having cancer were genotyped. The term "genotyped" as used herein refers to a process for determining a genotype of one or more subjects, where a "genotype" is a representation of one or more polymorphic variants in a population. Genotypes may be expressed in terms of a "haplotype," which as used herein refers to two or more polymorphic variants occurring within genomic DNA in a group of subjects within a population. For example, two SNPs may exist within a gene where each SNP position includes a cytosine variation and an adenine variation. Certain subjects in a population may carry one allele (heterozygous) or two alleles (homozygous) having the gene with a cytosine at each SNP position.

The present disclosure provides for both prophylactic and therapeutic methods of treating a subject having, or at risk of having cancer or other conditions that are treatable with tubulin stabilisation agents.

The term "effective amount" refers to a dosage or amount that is sufficient to reduce, halt, or slow tumor progression to result in alleviation, lessening or amelioration of symptoms in a patient or to achieve a desired biological outcome, e.g., slow or stop tumor growth or reduction or disappearance of a tumor.

"Pharmaceutically acceptable excipients or vehicles" include, for example, water, saline, glycerol, ethanol, etc. Additionally, auxiliary substances, such as wetting or emulsifying agents, pH buffering substances, and the like, may be present in such vehicles.

The therapeutic methods described generally comprise administration of a therapeutically effective amount of a tubulin stabilization agent, e.g., a modulator, e.g., an inhibitor or activator, to a subject in need of such treatment, such as a mammal, and particularly a primate such as a human. Treatment methods described also comprise administration of an effective amount of docetaxel to a subject, particularly a mammal such as a human in need of such treatment for an indication disclosed herein.

A variety of tubulin stabilization agents can be employed in the present treatment methods. Simple testing, e.g., in a standard anti-neoplastic assay can readily identify suitable tubulin stabilization agents. Suitable agents include those disclosed in US Patent Nos: 4814470, 5438072, 5698582, and 5714512.

Suitable compounds above and other tubulin stabilization agents can be readily prepared by known procedures or can be obtained from commercial sources. See, for example, Abe, A. et al., (1992) J. Biochem. 111:191-196; Inokuchi, J. et al. (1987) J. Lipid Res. 28:565-571; Shukla, A. et al. (1991) J. Lipid Res. 32:73; Vunnam, R.R. et al., (1980) Chem. and Physics of Lipids 26:265; Carson, K. et al., (1994) Tetrahedron Lets. 35:2659; and Akira, A. et al., (1995) J. Lipid Research 36:611.

In the therapeutic methods, a treatment compound can be administered to a subject in any of several ways. For example, a tubulin stabilization agent can be administered as a prophylactic to prevent the onset of or reduce the severity of a targeted condition. Alternatively, a tubulin stabilization agent can be administered during the course of a targeted condition.

In other therapeutic methods, provided are methods of treating a subject suffering from cancer, comprising determining a CYP1B1 genotype status of a subject or a cell of a subject, and administering a tubulin stabilization agent to the subject. The genotype status may be determined as described herein.

A treatment compound can be administered to a subject, either alone or in combination with one or more therapeutic agents, as a pharmaceutical composition in mixture with conventional excipient, e.g., pharmaceutically acceptable organic or inorganic carrier substances suitable for parenteral, enteral or intranasal application which do not deleteriously react with the active compounds and are not deleterious to the recipient thereof. Suitable pharmaceutically acceptable carriers include for example, water, salt solutions, alcohol, vegetable oils, polyethylene glycols, gelatin, lactose, amylose, magnesium stearate, talc, silicic acid, viscous paraffin, perfume oil, fatty acid monoglycerides and diglycerides, petroethral fatty acid esters, hydroxymethyl-cellulose, polyvinylpyrrolidone, etc. The pharmaceutical preparations can be sterilized and if desired mixed with auxiliary agents, e.g., lubricants, preservatives, stabilizers, wetting agents, emulsifiers, salts for influencing osmotic pressure, buffers, colorings, flavorings and/or aromatic substances and the like which do not deleteriously react with the active compounds.

Such compositions may be prepared for use in parenteral administration, particularly in the form of liquid solutions or suspensions; for oral administration, particularly in the form of tablets or capsules; intranasally, particularly in the form of powders, nasal drops, or aerosols; vaginally; topically e.g. in the form of a cream; rectally e.g. as a suppository; etc. The tubulin stabilization agents or activators may also be administered via stent. Exemplary stents are described in US Patent Application Publication Nos: 20050177246; 20050171599, 20050171597, 20050171598, 20050169969, 20050165474, 20050163821, 20050165352, and 20050171593.

The pharmaceutical agents may be conveniently administered in unit dosage form and may be prepared by any of the methods well known in the pharmaceutical arts, e.g., as described in Remington's Pharmaceutical Science (Mack Pub. Co., Easton, PA, 1980). Formulations for parenteral administration may contain as common excipients such as sterile water or saline, polyalkylene glycols such as polyethylene glycol, oils of vegetable origin, hydrogenated naphthalenes and the like. In particular, biocompatible, biodegradable lactide polymer, lactide/glycolide copolymer, or polyoxyethylene-polyoxypropylene copolymers may be useful excipients to control the release of certain tubulin stabilization agents.

Other potentially useful parenteral delivery systems include ethylene-vinyl acetate copolymer particles, osmotic pumps, implantable infusion systems, and liposomes. Formulations for inhalation administration contain as excipients, for example, lactose, or may be aqueous solutions containing, for example, polyoxyethylene-9-lauryl ether, glycocholate and deoxycholate, or oily solutions for administration in the form of nasal drops, or as a gel to be applied intranasally. Formulations for parenteral administration may also include glycocholate for buccal administration, methoxysalicylate for rectal administration, or citric acid for vaginal administration. Other delivery systems will administer the therapeutic agent(s) directly at a surgical site, e.g. after balloon angioplasty a tubulin stabilization agent may be administered by use of stents.

A tubulin stabilization agent (e.g., composition that promotes the assembly of tubulin, does not allow the disassembly of microtubules, binds to tubulin, etc) can be employed in the present treatment methods as the sole active pharmaceutical agent or can be used in combination with other active ingredients, e.g., anti-neoplastic or other compounds.

The concentration of one or more treatment compounds in a therapeutic composition will vary depending upon a number of factors, including the dosage of the tubulin stabilization agent to be administered, the chemical characteristics (e.g., hydrophobicity) of the composition employed, and the intended mode and route of administration. In general terms, one or more than one of the tubulin stabilization agents or activators may be provided in an aqueous physiological buffer solution containing about 0.1 to 10% w/v of a compound for parenteral administration.

It will be appreciated that the actual preferred amounts of active compounds used in a given therapy will vary according to e.g. the specific compound being utilized, the particular composition formulated, the mode of administration and characteristics of the subject, e.g. the species, sex, weight, general health and age of the subject. Optimal administration rates for a given protocol of administration can be readily ascertained by those skilled in the art using conventional dosage determination tests conducted with regard to the foregoing guidelines. Suitable dose ranges may include from about 1 µg/kg to about 100mg/kg of body weight per day.

Therapeutic compounds are suitably administered in a protonated and water-soluble form, e.g., as a pharmaceutically acceptable salt, typically an acid addition salt such as an inorganic acid addition salt, e.g., a hydrochloride, sulfate, or phosphate salt, or as an organic acid addition salt such as an acetate, maleate, fumarate, tartrate, or citrate salt. Pharmaceutically acceptable salts of therapeutic compounds also can include metal salts, particularly alkali metal salts such as a sodium salt or potassium salt; alkaline earth metal salts such as a magnesium or calcium salt; ammonium salts such an ammonium or tetramethyl ammonium salt; or an amino acid addition salts such as a lysine, glycine, or phenylalanine salt.

Preferred tubulin stabilization agents exhibit significant activity in a standard cell proliferation assays when the cells used in the assays are homozygous wild-type CYP1B1 or heterozygous wild-type/CYP1B1*3 (e.g., CYP1B1*1/*3). Preferably, the tubulin stabilization agent inhibits cell proliferation by at least 15 or 25%, preferably at least 50%, relative to a suitable control assay. In such an assay, between about 0.1 to 100µM, preferably between about 1 to 50µM of a desired tubulin stabilization agent is used. Exemplary cell proliferation assays include counting viable cells and monitoring activity of specified citric acid cycle enzymes such as lactate dehydrogenase.

One assay measures incorporation of one or more detectably-labeled nucleosides into DNA, e.g., by:
a) culturing suitable cells in medium and adding
   1) a candidate tubulin stabilization agent and
   2) a radiolabeled nucleoside such as 3H- thymidine typically in an amount between about 0.1 to 100µCi;
b) incubating the cells, e.g., for about 6-24 hours, and typically followed by washing; and
c) measuring incorporation of the radiolabeled nucleoside into DNA over that time relative to a control culture that is prepared and incubated under the same conditions as the assay culture but does not include the potential tubulin stabilization agent. The measurement can be achieved by several methods including trichloroacetic acid (TCA) precipitation of labeled DNA on filters followed by scintillation counting. See e.g., Chatterjee, S., Biochem. Biophys. Res Comm. (1991) 181:554; Chatterjee, S. et al. (1982) Eur. J. Biochem. 120:435 for disclosure relating to this assay.

References herein to a "standard *in vitro* cell proliferation assay" or other similar phrase refer to an assay that includes the above steps a) through c). One preferred example of a cell proliferation assay uses tumor cells, particularly those obtained from a human, cow or a rabbit. A suitable protocol involves preparing tumor cells according to standard methods and culturing same in microtitre plates in a suitable medium. A desired tubulin stabilization agent is then diluted in the medium, preferably to a final concentration of between about 1 to 100µg, more preferably between about to 50µg per ml of medium or less followed by an incubation period of between about 1-5 days, preferably about 1 day or less. Following the incubation, a standard cell proliferation can be conducted, e.g., incorporation of tritiated thymidine or lactate dehydrogenase assay as mentioned above. The assays are preferably conducted in triplicate with a variation of between 5% to 10%. See e.g., Ross, R. J. Cell. Biol. (1971) 50:172; Chatterjee, S. et al. (1982) Eur. J. Biochem. 120:435; Bergmeyer, H.V. In Principles of Enzymatic Analysis. (1978) Verlag Chemie, NY.

Methods for determining the therapeutic capacity of a tubulin stabilization agent to reduce, halt, or otherwise modify tumor growth in a subject comprise determining pre-treatment levels or size of tumor masses in a subject; administering a therapeutically effective amount of a tubulin stabilization agent to the subject; and determining a post-treatment levels or size of tumor masses in subject. In one embodiment, a decrease in the tumor size indicated that the tubulin stabilization agent is efficacious. In a related embodiment, the stabilization of tumor size in a subject indicates that the agent is efficacious.

Method for determining the therapeutic capacity of a candidate tubulin stabilization agent for treating cancer may also comprise providing a population of tumor cells with a known CYP1B1 genotype; contacting the cells with a candidate composition; and determining effect of the candidate composition on cell proliferation, wherein a decrease in cell proliferation indicates that the candidate composition may be efficacious.

Method for determining the therapeutic capacity of a tubulin stabilization agent to reduce tissue degeneration in a subject, may further comprise determining a CYP1B1 genotype status of a subject or a cell of a subject; determining a pre-treatment tumor status in the subject; administering a therapeutically effective amount of a tubulin stabilization agent to the subject; and determining a post-treatment tumor status in the subject.

A method of assessing the therapeutic capacity or efficacy of the treatment in a subject includes determining the pre-treatment tumor status (e.g., by visual inspection of tissue, measurement of tumor regression or growth at various times before, during and after treatment, wherein the measurement is with, for example, a caliper) and then administering a therapeutically effective amount of a tubulin stabilization agent to the subject. After an appropriate period of time (e.g., after an initial period of treatment) after the administration of the compound, e.g., 2 hours, 4 hours, 8 hours, 12 hours, or 72 hours, the level of tumor growth or cell proliferation is determined again. The modulation of the cell proliferation indicates efficacy of the treatment. The tumor status may be determined periodically throughout treatment. For example, the tumor status may be checked every few hours, days or weeks to assess the further efficacy of the treatment. A decrease in tumor growth, for example, indicates that the treatment with an agent is efficacious. The method described may be used to screen or select a subject or a compound that may benefit from treatment with a tubulin stabilization agent or may be an effective agent, respectively.

A control experiment is generally tailored for use in a particular assay. For example, most control experiments involve subjecting a test sample (e.g., a population of cells or lysate thereof) to medium, saline, buffer or water instead of a potential tubulin stabilization agent in parallel to the cells receiving an amount of test compound. A desired assay is then conducted in accordance with the present methods.

The methods described herein and used to develop the methods here can utilize or utilized a variety of different informative comparisons to identify correlations. For example a plurality of pairwise comparisons of treatment response and the presence or absence of at least one variance can be performed for a plurality of patients. Likewise, the method can involve comparing the response of at least one patient homozygous for at least one variance with at least one patient homozygous for the alternative form of that variance or variances. The method can also involve comparing the response of at least one patient heterozygous for at least one variance with the response of at least one patient homozygous for the at least one variance; Preferably the heterozygous patient response is compared to both alternative homozygous forms, or the response of heterozygous patients is grouped with the response of one class of homozygous patients and said group is compared to the response of the alternative homozygous group.

By "prediction of patient outcome" is meant a forecast of the patient's likely health status. This may include a prediction of the patient's response to therapy, rehabilitation time, recovery time, cure rate, rate of disease progression, predisposition for future disease, or risk of having relapse.

By "therapy for the treatment of a disease" is meant any pharmacological agent or drug with the property of healing, curing, or ameliorating any symptom or disease mechanism associated with drug-induced disease, disorder or dysfunction.

By "responder population" is meant a patient or patients that respond favorably to a given therapy.

By "pathway" or "gene pathway" is meant the group of biologically relevant genes involved in a pharmacodynamic or pharmacokinetic mechanism of drug, agent, or candidate therapeutic intervention. These mechanisms may further include any physiologic effect the drug or candidate therapeutic intervention renders. Included in this are "biochemical pathways" which is used in its usual sense to refer to a series of related biochemical processes (and the corresponding genes and gene products) involved in carrying out a reaction or series of reactions. Generally in a cell, a pathway performs a significant process in the cell.

By "pharmacological activity" used herein is meant a biochemical or physiological effect of drugs, compounds, agents, or candidate therapeutic interventions upon administration and the mechanism of action of that effect.

The pharmacological activity is then determined by interactions of drugs, compounds, agents, or candidate therapeutic interventions, or their mechanism of action, on their target proteins or macromolecular components. By "agonist" or "mimetic" or "activators" is meant a drug, agent, or compound that activate physiologic components and mimic the effects of endogenous regulatory compounds. By "antagonist," "blockers" or "inhibitors" is meant drugs, agents, or compounds that bind to physiologic components and do not mimic endogenous regulatory compounds, or interfere with the action of endogenous regulatory compounds at physiologic components. These inhibitory compounds do not have intrinsic regulatory activity, but prevent the action of agonists. By "partial agonist" or "partial antagonist" is meant an agonist or antagonist, respectively, with limited or partial activity. By "negative agonist" or "inverse antagonists" is meant that a drug, compound, or agent that can interact with a physiologic target protein or macromolecular component and stabilizes the protein or component such that agonist-dependent conformational changes of the component do not occur and agonist mediated mechanism of physiological action is prevented. By "modulators" or "factors" is meant a drug, agent, or compound that interacts with a target protein or macromolecular component and modifies the physiological effect of an agonist.

### Pharmaceutical Compositions

The small molecule, peptide, nucleic acid, and antibody therapeutics described herein may be formulated into pharmaceutical compositions and be provided in kits. The pharmaceutical formulations may also be coated on medical devices or onto nano-particles for delivery.

The phrase "pharmaceutically acceptable carrier" is art recognized and includes a pharmaceutically acceptable material, composition or vehicle, suitable for administering compound to mammals. The carriers include liquid or solid filler, diluent, excipient, solvent or encapsulating material, involved in carrying or transporting the subject agent from one organ, or portion of the body, to another organ, or portion of the body. Each carrier must be "acceptable" in the sense of being compatible with the other ingredients of the formulation and not injurious to the patient. Some examples of materials which can serve as pharmaceutically acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; phosphate buffer solutions; and other non-toxic compatible substances employed in pharmaceutical formulations.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically acceptable antioxidants include: water soluble antioxidants, such as ascorbic acid, cysteine hydrochloride, sodium bisulfate, sodium metabisulfite, sodium sulfite and the like; oil-soluble antioxidants, such as ascorbyl palmitate, butylated hydroxyanisole (BHA), butylated hydroxytoluene (BHT), lecithin, propyl gallate, .alpha.-tocopherol, and the like; and metal chelating agents, such as citric acid, ethylenediamine tetraacetic acid (EDTA), sorbitol, tartaric acid, phosphoric acid, and the like.

Formulations include those suitable for oral, nasal, topical, transdermal, buccal, sublingual, intramuscular, intraperotineal, rectal, vaginal and/or parenteral administration. The formulations may conveniently be presented in unit dosage form and may be prepared by any methods well known in the art of pharmacy. The amount of active ingredient that can be combined with a carrier material to produce a single dosage form will generally be that amount of the compound that produces a therapeutic effect. Generally, out of one hundred percent, this amount will range from about 1 percent to about ninety-nine percent of active ingredient, preferably from about 5 percent to about 70 percent, most preferably from about 10 percent to about 30 percent.

Methods of preparing these formulations or compositions include the step of bringing into association an antibody or complex with the carrier and, optionally, one or more accessory ingredients. In general, the formulations are prepared by uniformly and intimately bringing into association a compound with liquid carriers, or finely divided solid carriers, or both, and then, if necessary, shaping the product.

Formulations suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound as an active ingredient. A compound may also be administered as a bolus, electuary or paste.

In solid dosage forms for oral administration (capsules, tablets, pills, dragees, powders, granules and the like), the active ingredient is mixed with one or more pharmaceutically acceptable carriers, such as sodium citrate or dicalcium phosphate, and/or any of the following: fillers or extenders, such as starches, lactose, sucrose, glucose, mannitol, and/or silicic acid; binders, such as, for example, carboxymethylcellulose, alginates, gelatin, polyvinyl pyrrolidone, sucrose and/or acacia; humectants, such as glycerol; disintegrating agents, such as agar-agar, calcium carbonate, potato or tapioca starch, alginic acid, certain silicates, and sodium carbonate; solution retarding agents, such as paraffin; absorption accelerators, such as quaternary ammonium compounds; wetting agents, such as, for example, cetyl alcohol and glycerol monostearate; absorbents, such as kaolin and bentonite clay; lubricants, such a talc, calcium stearate, magnesium stearate, solid polyethylene glycols, sodium lauryl sulfate, and mixtures thereof; and coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered compound moistened with an inert liquid diluent.

The tablets, and other solid dosage forms of the pharmaceutical compositions, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions that can be used include polymeric substances and waxes. The active ingredient can also be in micro-encapsulated form, if appropriate, with one or more of the above-described excipients.

Liquid dosage forms for oral administration of the compounds include pharmaceutically acceptable emulsions, microemulsions, solutions, suspensions, syrups and elixirs. In addition to the active ingredient, the liquid dosage forms may contain inert diluent commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert dilutents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations of the pharmaceutical compositions for rectal or vaginal administration may be presented as a suppository, which may be prepared by mixing one or more compounds with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active compound.

Formulations which are suitable for vaginal administration also include pessaries, tampons, creams, gels, pastes, foams or spray formulations containing such carriers as are known in the art to be appropriate.

Dosage forms for the topical or transdermal administration of a compound include powders, sprays, ointments, pastes, creams, lotions, gels, solutions, patches and inhalants. The active compound may be mixed under sterile conditions with a pharmaceutically acceptable carrier, and with any preservatives, buffers, or propellants that may be required.

The ointments, pastes, creams and gels may contain, in addition to an active compound, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to a compound described, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

Transdermal patches have the added advantage of providing controlled delivery of a compound described to the body. Such dosage forms can be made by dissolving or dispersing the compound in the proper medium. Absorption enhancers can also be used to increase the flux of the compound across the skin. The rate of such flux can be controlled by either providing a rate controlling membrane or dispersing the active compound in a polymer matrix or gel.

Ophthalmic formulations, eye ointments, powders, solutions and the like, are also contemplated.

Pharmaceutical compositions suitable for parenteral administration comprise one or more compounds described in combination with one or more pharmaceutically acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and nonaqueous carriers that may be employed in the pharmaceutical compositions include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganism may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents that delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution which, in turn, may depend upon crystal size and crystalline form. Alternatively, delayed absorption of a parenterally-administered drug form is accomplished by dissolving or suspending the drug in an oil vehicle.

Injectable depot forms are made by forming microencapsule matrices of the subject compounds in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions that are compatible with body tissue.

The preparations may be given orally, parenterally, topically, or rectally. They are of course given by forms suitable for each administration route. For example, they are administered in tablets or capsule form, by injection, inhalation, eye lotion, ointment, suppository, etc. administration by injection, infusion or inhalation; topical by lotion or ointment; and rectal by suppositories. Oral administration is preferred.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion.

The phrases "systemic administration," "administered systemically," "peripheral administration" and "administered peripherally" as used herein mean the administration of a compound, drug or other material other than directly into the central nervous system, such that it enters the patient's system and, thus, is subject to metabolism and other like processes, for example, subcutaneous administration.

The compounds may be administered to humans and other animals for therapy by any suitable route of administration, including orally, nasally, as by, for example, a spray, rectally, intravaginally, parenterally, intracisternally and topically, as by powders, ointments or drops, including buccally and sublingually.

Regardless of the route of administration selected, the compounds described, which may be used in a suitable hydrated form, and/or the pharmaceutical compositions, are formulated into pharmaceutically acceptable dosage forms by conventional methods known to those of skill in the art.

Actual dosage levels of the active ingredients in the pharmaceutical compositions may be varied so as to obtain an amount of the active ingredient which is effective to achieve the desired therapeutic response for a particular patient, composition, and mode of administration, without being toxic to the patient.

The selected dosage level will depend upon a variety of factors including the activity of the particular compound employed, or the ester, salt or amide thereof, the route of administration, the time of administration, the rate of excretion of the particular compound being employed, the duration of the treatment, other drugs, compounds and/or materials used in combination with the particular compound employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the compounds employed in the pharmaceutical composition at levels lower than that required in order to achieve the desired therapeutic effect and gradually increase the dosage until the desired effect is achieved.

In general, a suitable daily dose of a compound described will be that amount of the compound that is the lowest dose effective to produce a therapeutic effect. Such an effective dose will generally depend upon the factors described above. Generally, intravenous and subcutaneous doses of the compounds for a patient, when used for the indicated analgesic effects, will range from about 0.0001 to about 100 mg per kilogram of body weight per day, more preferably from about 0.01 to about 50 mg per kg per day, and still more preferably from about 1.0 to about 100 mg per kg per day. An effective amount is that amount that treats cancer or associated disease.

If desired, the effective daily dose of the active compound may be administered as one dose or as, two, three, four, five, six or more sub-doses administered separately at appropriate intervals throughout the day, optionally, in unit dosage forms.

While it is possible for a compound described to be administered alone, it is preferable to administer the compound as a pharmaceutical composition. Moreover, the pharmaceutical compositions described herein may be administered with one or more other active ingredients that would aid in treating a subject having a HIV infection. In a related aspect, the pharmaceutical compositions may be formulated to contain one or more additional active ingredients that would aid in treating a subject having a HIV infection or associated disease or disorder.

The antibodies and complexes, produced as described above, can be provided in kits, with suitable instructions and other necessary reagents, in order to conduct immunoassays as described above. The kit can also contain, depending on the particular immunoassay used, suitable labels and other packaged reagents and materials (e.g., wash buffers and the like). Standard immunoassays, such as those described above, can be conducted using these kits. The pharmaceutical compositions can be included in a container, pack, kit or dispenser together with instructions, e.g., written instructions, for administration, particularly such instructions for use of the antibody or complex to treat or prevent cancer or associated disease. The container, pack, kit or dispenser may also contain, for example, one or more additional active ingredients that would aid in treating a subject having aberrant cell proliferation.

The therapeutic agents described herein, e.g., tubulin stabilization agents, are formulated into pharmaceutical preparations for administration.

Additional therapeutic agents may include, but are not limited to, immunomodulatory agents, anti-inflammatory agents (*e.g.,* adrenocorticoids, corticosteroids (*e.g.,* beclomethasone, budesonide, flunisolide, fluticasone, triamcinolone, methlyprednisolone, prednisolone, prednisone, hydrocortisone), glucocorticoids, steroids, non-steriodal anti-inflammatory drugs (*e.g.,* aspirin, ibuprofen, diclofenac, and COX-2 inhibitors), and leukotreine antagonists (*e.g.,* montelukast, methyl xanthines, zafirlukast, and zileuton), beta2-agonists (e.g., albuterol, biterol, fenoterol, isoetharie, metaproterenol, pirbuterol, salbutamol, terbutalin formoterol, salmeterol, and salbutamol terbutaline), anticholinergic agents (e.g., ipratropium bromide and oxitropium bromide), sulphasalazine, penicillamine, dapsone, antihistamines, anti-malarial agents (e.g., hydroxychloroquine), anti-viral agents, and antibiotics (e.g., dactinomycin (formerly actinomycin), bleomycin, erythomycin, penicillin, mithramycin, anthramycin (AMC))

### Antibodies

Antibodies useful in the methods described herein are antibodies specific for and can distinguish alleles of CYP1B1, for example, can distinguish between CYP1B1 wild-type and CYP1B1*3. Methods of generating antibodies useful in the methods described herein are described more fully below.

Chimeric and humanized monoclonal antibodies, comprising both human and non-human portions, can be made using standard recombinant DNA techniques. Such chimeric and humanized monoclonal antibodies can be produced by recombinant DNA techniques known in the art, for example using methods described in Robinson et al. International Application No. PCT/US86/02269; Akira, et al. European Patent Application 184,187; Taniguchi, M., European Patent Application 171,496; Morrison et al. European Patent Application 173,494; Neuberger et al. PCT International Publication No. WO 86/01533; Cabilly et al. U.S. Pat. No. 4,816,567; Cabilly et al. European Patent Application 125,023; Better et al. (1988) Science 240: 1041-1043; Liu et al. (1987) Proc. Natl. Acad. Sci. USA 84: 3439-3443; Liu et al. (1987) J. Immunol. 139: 3521-3526; Sun et al. (1987) Proc. Natl. Acad. Sci. USA 84: 214-218; Nishimura et al. (1987) Canc. Res. 47: 999-1005; Wood et al. (1985) Nature 314: 446-449; and Shaw et al. (1988) J. Natl. Cancer Inst. 80: 1553-1559); Morrison, S. L. (1985) Science 229: 1202-1207; Oi et al. (1986) BioTechniques 4: 214; Winter U.S. Pat. No. 5,225,539; Jones et al. (1986) Nature 321: 552-525; Verhoeyan et al. (1988) Science 239: 1534; and Beidler et al. (1988) J. Immunol. 141: 4053-4060.

Completely human antibodies are particularly desirable for therapeutic treatment of human patients. Such antibodies can be produced using transgenic mice that are incapable of expressing endogenous immunoglobulin heavy and light chains genes, but which can express human heavy and light chain genes. See, for example, Lonberg and Huszar (1995) Int. Rev. Immunol. 13: 65-93); and U.S. Pat. Nos. 5,625,126; 5,633,425; 5,569,825; 5,661,016; and 5,545,806. In addition, companies such as Abgenix, Inc. (Fremont, Calif.) and Medarex, Inc. (Princeton, N.J.), can be engaged to provide human antibodies directed against a selected antigen using technology similar to that described above.

Completely human antibodies that recognize a selected epitope can be generated using a technique referred to as "guided selection." In this approach a selected non-human monoclonal antibody, e.g., a murine antibody, is used to guide the selection of a completely human antibody recognizing the same epitope. This technology is described by Jespers et al. (1994) Bio/Technology 12: 899-903).

The term "monoclonal antibody" as used herein refers to an antibody obtained from a substantially homogeneous population of antibodies, e.g., the subject antibodies within the population are identical except for possible naturally occurring mutations that may be present in a small subset of the antibody molecules. The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, as long as they exhibit the desired antagonistic activity (See, U.S. Pat. No. 4,816,567; and Morrison et al., Proc. Natl. Acad. Sci. USA 81: 6851-6855 (1984)).

The present monoclonal antibodies can be made using any procedure which produces monoclonal antibodies. For example, monoclonal antibodies can be prepared using hybridoma methods, such as those described by Kohler and Milstein, Nature, 256: 495 (1975). In a hybridoma method, a mouse or other appropriate host animal is typically immunized with an immunizing agent to elicit lymphocytes that produce antibodies that will specifically bind to the immunizing agent.

The monoclonal antibodies also can be made by recombinant DNA methods, such as those described in U.S. Pat. No. 4,816,567 (Cabilly et al.). DNA encoding the disclosed monoclonal antibodies can be readily isolated and sequenced using conventional procedures (e.g., by using oligonucleotide probes that are capable of binding specifically to genes encoding the heavy and light chains of antibodies). Libraries of antibodies or active antibody fragments also can be generated and screened using phage display techniques, e.g., as described in U.S. Pat. No. 5,804,440 to Burton et al. and U.S. Pat. No. 6,096,551 to Barbas et al.

In vitro methods are also suitable for preparing monovalent antibodies. Digestion of antibodies to produce fragments thereof, particularly, Fab fragments, can be accomplished using routine techniques known in the art. For instance, digestion can be performed using papain. Examples of papain digestion are described in International Patent Application Publication No. WO 94/29348, published Dec. 22, 1994, and U.S. Pat. No. 4,342,566. Papain digestion of antibodies typically produces two identical antigen binding fragments, called Fab fragments, each with a single antigen binding site, and a residual Fc fragment. Pepsin treatment yields a fragment that has two antigen combining sites and is still capable of cross-lining antigen.

As used herein, the term "antibody or fragments thereof" encompasses chimeric antibodies and hybrid antibodies, with dual or multiple antigen or epitope specificities, single chain antibodies and fragments, such as F(ab')2, Fab', Fab, scFv and the like, including hybrid fragments. Thus, fragments of the antibodies that retain the ability to bind their specific antigens are provided. For example, fragments of antibodies which maintain HIV gp 120 binding activity are included within the meaning of the term "antibody or fragment thereof." Such antibodies and fragments can be made by techniques known in the art and can be screened for specificity and activity according to the methods set forth in the Examples and in general methods for producing antibodies and screening antibodies for specificity and activity (See Harlow and Lane. Antibodies, A Laboratory Manual. Cold Spring Harbor Publications, New York (1988)). Also included within the meaning of "antibody or fragments thereof" are conjugates of antibody fragments and antigen binding proteins (single chain antibodies) as described, for example, in U.S. Pat. No. 4,704,692.

The fragments, whether attached to other sequences or not, can also include insertions, deletions, substitutions, or other selected modifications of particular regions or specific amino acids residues, provided the activity of the antibody or antibody fragment is not significantly altered or impaired compared to the non-modified antibody or antibody fragment. These modifications can provide for some additional property, such as to remove/add amino acids capable of disulfide bonding, to increase bio-longevity, to alter secretory characteristics; etc. In any case, the antibody or antibody fragment must possess a bioactive property, such as specific binding to its cognate antigen. Functional or active regions of the antibody or antibody fragment can be identified by mutagenesis of a specific region of the protein, followed by expression and testing of the expressed polypeptide. Such methods are readily apparent to a skilled practitioner in the art arid can include site-specific mutagenesis of the nucleic acid encoding the antibody or antibody fragment (Zoller, M. J. Curr. Opin. Biotechnol. 3: 348-354 (1992)).

As used herein, the term "antibody" or "antibodies" can also refer to a human antibody and/or a humanized antibody. Many non-human antibodies (e.g., those derived from mice, rats, or rabbits) are naturally antigenic in humans, and thus can give rise to undesirable immune responses when administered to humans. Therefore, the use of human or humanized antibodies in the methods of the invention serves to lessen the chance that an antibody administered to a human will evoke an undesirable immune response.

Human antibodies also can be prepared using any other technique. Examples of techniques for human monoclonal antibody production include those described by Cole et al. (Monoclonal Antibodies and Cancer Therapy, Alan R. Liss, p. 77 (1985)) and by Boerner et al. (J. Immunol. 147(1): 86-95 (1991)). Human antibodies (and fragments thereof) also can be produced using phage display libraries (Hoogenboom et al., J. Mol. Biol. 227: 381 (1991); Marks et al., J. Mol. Biol. 222: 581 (1991)).

Human antibodies also can be obtained from transgenic animals. For example, transgenic, mutant mice that can produce a full repertoire of human antibodies in response to immunization have been described (see, e.g., Jakobovits et al., Proc. Natl. Acad Sci. USA 90: 2551-255 (1993); Jakobovits et al., Nature 362: 255-258 (1993); and Bruggermann et al., Year in Immunol. 7: 33 (1993)). Specifically, the homozygous deletion of the antibody heavy chain joining region (J(H) gene in these chimeric and germ-line mutant mice results in complete inhibition of endogenous antibody production, and the successful transfer of the human germ-line antibody gene array into such germ-line mutant mice results in the production of human antibodies upon antigen challenge.

Antibody humanization techniques generally involve the use of recombinant DNA technology to manipulate the DNA sequence encoding one or more polypeptide chains of an antibody molecule. Accordingly, a humanized form of a non-human antibody (or a fragment thereof) is a chimeric antibody or antibody chain (or a fragment thereof, such as an Fv, Fab, Fab', or other antigen-binding portion of an antibody) which contains a portion of an antigen binding site from a non-human (donor) antibody integrated into the framework of a human (recipient) antibody.

To generate a humanized antibody, residues from one or more complementarity determining regions (CDRs) of a recipient (human) antibody molecule are replaced by residues from one or more CDRs of a donor (non-human) antibody molecule that is known to have desired antigen binding characteristics (e.g., a certain level of specificity and affinity for the target antigen). In some instances, Fv framework (FR) residues of the human antibody are replaced by corresponding non-human residues. Humanized antibodies may also contain residues which are found neither in the recipient antibody nor in the imported CDR or framework sequences. Generally, a humanized antibody has one or more amino acid residues introduced into it from a source which is non-human. In practice, humanized antibodies are typically human antibodies in which some CDR residues and possibly some FR residues are substituted by residues from analogous sites in rodent antibodies. Humanized antibodies generally contain at least a portion of an antibody constant region (Fc), typically that of a human antibody (Jones et al., Nature 321: 522-525 (1986); Reichmann et al., Nature 332: 323-327 (1988); and Presta, Curr. Opin. Struct. Biol. 2: 593-596 (1992)).

Methods for humanizing non-human antibodies are well-known in the art. For example, humanized antibodies can be generated according to the methods of Winter and co-workers (Jones et al., Nature 321: 522-525 (1986); Riechmann et al., Nature 332: 323-327 (1988); and Verhoeyen et al., Science 239: 1534536 (1988)), by substituting rodent CDRs or CDR sequences for the corresponding sequences of a human antibody. Methods that can be used to produce humanized antibodies are also described in U.S. Pat. No. 4,816,567 (Cabilly et al.), U.S. Pat. No. 5,565,332 (Hoogenboom et al.), U.S. Pat. No. 5,721,367 (Kay et al.), U.S. Pat. No. 5,837,243 (Deo et al.), U.S. Pat. No. 5,939,598 (Kucherlapati et al.), U.S. Pat. No. 6,130,364 (Jakobovits et al.), and U.S. Pat. No. 6,180,377 (Morgan et al.).

### KITS

In one aspect, kits for the assessment of cancer treatment-options are provided. The kits comprise oligonucleotide probes that differentiate the wild-type and variant alleles of CYP1B1, wherein the allele nucleotide position 4326. Optionally the kits contain instructions for use.

The oligonucleotide probes may be one or more of OLA, or Taqman.

The kits may comprise oligonucleotide primes that amplify from about nt 4300 to about nt 4350 portion of CYP1B1 and instructions for use. The primers may be labeled.

In another aspect, kits for the assessment of cancer treatment options are provided and comprise an array and/or microarray, oligonucleotide primes that amplify from about nucleotide 4300 to about nucleotide 4350 portion of CYP1B1 and instructions for use. Alternately or in addition, primers may be provided that amplify from about nucleotide 4000 to about nucleotide 4500 of CYP1B1, from about nucleotide 4100 to about nucleotide 4400 of CYP1B1, from about nucleotide 4200 to about nucleotide 4400 of CYP1B1, from about nucleotide 4250 to about nucleotide 4375 of CYP1B1, or other portion that one of skill in the art would determine necessary or adequate to amplify and detect the genotype status using array or microarray technology.

In another aspect, a kit for the assessment of cancer treatment options are provided and comprise antibodies that distinguish the wild type and variant (e.g., CYP1B1*3) alleles.

Optionally the kits may comprise instructions for use.

The kits described above may further contain enzymes, buffers, labeling agents, and/or pharmaceutical compositions for treatment.

Another aspect provides a kit containing at least one probe or at least one primer (or other amplification oligonucleotide) or both (e.g., as described above) corresponding to CYP1B1 or other gene related to a drug-induced disease or condition, or other gene involved in absorption, distribution, metabolism, excretion, or in toxicity-related modification of a drug. The kits are preferably adapted and configured to be suitable for identification of the presence or absence of a particular variance or variances, which can include or consist of a nucleic acid sequence corresponding to a portion of a gene. A plurality of variances may comprise a haplotype of haplotypes. The kit may also contain a plurality of either or both of such probes and/or primers, e.g., 2, 3, 4, 5, 6, or more of such probes and/or primers. Preferably the plurality of probes and/or primers are adapted to provide detection of a plurality of different sequence variances in a gene or plurality of genes, e.g., in 2, 3, 4, 5, or more genes or to amplify and/or sequence a nucleic acid sequence including at least one variance site in a gene or genes. Preferably one or more of the variance or variances to be detected are correlated with variability in a treatment response or tolerance, and are preferably indicative of an effective response to a treatment. In preferred embodiments, the kit contains components (e.g., probes and/or primers) adapted or useful for detection of a plurality of variances (which may be in one or more genes) indicative of the effectiveness of at least one treatment, preferably of a plurality of different treatments for a particular disease or condition. It may also be desirable to provide a kit containing components adapted or useful to allow detection of a plurality of variances indicative of the effectiveness of a treatment or treatment against a plurality of diseases. The kit may also optionally contain other components, preferably other components adapted for identifying the presence of a particular variance or variances. Such additional components can, for example, independently include a buffer or buffers, e.g., amplification buffers and hybridization buffers, which may be in liquid or dry form, a DNA polymerase, e.g., a polymerase suitable for carrying out PCR (e.g., a thermostable DNA polymerase), and deoxy nucleotide triphosphates (dNTPs). Preferably a probe includes a detectable label, e.g., a fluorescent label, enzyme label, light scattering label, or other label. Preferably the kit includes a nucleic acid or polypeptide array on a solid phase substrate. The array may, for example, include a plurality of different antibodies, and/or a plurality of different nucleicacid sequences. Sites in the array can allow capture and/or detection of nucleic acid sequences or gene products corresponding to different variances in one or more different genes. Preferably the array is arranged to provide variance detection for a plurality of variances in one or more genes which correlate with the effectiveness of one or more treatments of one or more diseases, which is preferably a variance as described herein.

The kit may also optionally contain instructions for use, which can include a listing of the variances correlating with a particular treatment or treatments for a disease or diseases and/or a statement or listing of the diseases for which a particular variance or variances correlates with a treatment efficacy and/or safety.

Preferably the kit components are selected to allow detection of a variance described herein, and/or detection of a variance indicative of a treatment, e.g., administration of a drug, pointed out herein.

Additional configurations for kits will be apparent to those skilled in the art.

The invention includes the use of such a kit to determine the genotype(s) of one or more subjects with respect to one or more variance sites in one or more genes identified herein. Such use can include providing a result or report indicating the presence and/or absence of one or more variant forms or a gene or genes which are indicative of the effectiveness of a treatment or treatments.

**Supplemental Table 1. Genotype frequencies for the studied variants***

| Polymorphism | Ethnicity | N | WT^{†} | Het^{†} | Var^{†} |
|---|---|---|---|---|---|
| CYP1B1*2 | CA | 21 | 11 (52.3%) | 6 (28.5%) | 4 (19.0%) |
| | AA | 3 | 0 | 0 | 3 (100%) |
| | A | 1 | NA | NA | NA |
| | | | | | |
| CYP1B1*3 | CA | 21 | 6 (28.6%) | 12 (57.1%) | 3 (14.3%) |
| | AA | 3 | 0 | 1 (33.3%) | 2 (66.7%) |
| | A | 1 | 1 (100%) | 0 | 0 |
| | | | | | |
| CYP1B1*4 | CA | 21 | 14 (66.7%) | 6 (28.6%) | 1 (4.8%) |
| | AA | 3 | 3 (100%) | 0 | 0 |
| | A | 1 | 1 (100%) | 0 | 0 |

| | | | | | |
|---|---|---|---|---|---|
| *N, number of samples; CA = Caucasian American; AA = African American; A = Asian; NA = data not available. ^{†}Denotes number of patients with the specified CYP1B1 genotype for the various polymorphisms, with percentage of the entire group in parenthesis; WT, wild-type; Het, heterozygous variant; Var, Homozygous variant | | | | | |

**Table 1. Patient demographics by CYP1B1*3 genotype***

| Characteristic | All patients | WT and Het^{†} | Var^{†} |
|---|---|---|---|
| *Baseline screening* | | | |
| Total entered | 25 | 20 | 5 |
| Age (years) | 65.7 (42 - 81) | 67.5 (42 - 81) | 58.7 (50 - 70) |
| Body-surface area (m²) | 2.0 (1.6 - 2.5) | 2.0 (1.6 - 2.3) | 2.0 (1.8 - 2.5) |
| Gleason Score | 8 (6-9) | 8 (6 - 9)^{‡} | 8 (7 - 9) |
| ECOG performance status | | | |
| 0 | 5 | 5 | 0 |
| 1 | 20 | 15 | 5 |
| Pts with measurable soft tissue lesion(s) | 11 | 9 | 2 |
| | | | |

| *Pretherapy clinical chemistry* | | | |
|---|---|---|---|
| Prostate Specific Antigen (ng/mL) | 94.41 (0.2 - 340.8) | 78.63 (2.3 - 284.0 | 157-52 (0.2 - 340.8) |
| Hemoglobin (g/dL) | 12.83 (8.8-15.8) | 13.13 (10.2-15.8) | 11.67 (8.8-15.4) |
| Lactate Dehydrogenase (units/L) | 196.4 (116-296) | 196.7 (116-296) | 195.4 (152-295) |
| Alkaline phosphatase (units/L) | 148 (50-429) | 133.4 (50 - 295) | 207 (106-429) |
| Serum albumin (g/L) | 4.1 (3.3-4.8) | 4.2 (3.7-4.8) | 3.8 (33-4.3) |

| | | | |
|---|---|---|---|
| * Continuous data are given as median with range in parenthesis, and categorical data as number of patients with percentage of the total population in parentheses. ^{†}Denotes CYP1B1*3 genotype; WT, wild-type; Heterozygous variant; Var, Homozygous variant ^{‡}Gleason scores for two patients were unavailable | | | |

### EXAMPLES

The present invention is further illustrated by the following non-limiting examples.

### EXAMPLE 1

The CYP1B1 genotypes on germline DNA obtained from 25 men with AIPC treated with docetaxel (Aventis Pharmaceuticals, Bridgewater, NJ) were retrospectively evaluated. The subjects were administered docetaxel intravenously over 1-hour at a dose of 30 mg/m² in whom detailed survival data were available (Table 1). Inclusion and exclusion criteria have been previously published (12). The study protocol was approved by the Institutional Review Board (Bethesda, MD), and all patients provided written informed consent. DNA was isolated from whole blood and variants in the CYP1B1 gene were analyzed by direct nucleotide sequencing (see Legend to Figure 1). The association between genotypes and progression-free survival and overall survival was determined by the Kaplan-Meier method using a two-tailed log rank test. The homozygous wild-type (CYP1B1*1/*1) and heterozygous variant (CYP1B1*1/*3) genotypes were pooled in the analysis on the basis of similarity of outcomes. The associated p-value was adjusted to be two times the unadjusted value to account for the two implicit comparisons made in determining the desirability of combining groups. Progression was censored at the off study date if a patient was removed for toxicity or another reason.

CYP1B1*3 genotyping data were compared with previously reported genotype frequencies in a predominantly Caucasian population (13), and found to be similar (*P* = .69, chi-squared test) (Supplemental Table 1). It was found that the CYP1B1*3 polymorphism is a marker for a poor prognosis in patients who have undergone docetaxel treatment as the CYP1B1*3 polymorphism predicts a decrease in median survival time of 7.8 months (*P* = .012); the 7 subjects with the wild type sequence combined with 13 heterozygotes had a significantly longer overall median survival (15.3 months) compared to that of 5 patients who were homozygote variant (CYP1B1*3/*3) (7.5 months) (Figure 1A). The data also suggest a strong trend towards the CYP1B1*3/*3 variant being associated with a decreased progression free survival as compared to pooled wild type and heterozygous genotypes (3.3 months vs 6.2 months, respectively), although the difference was not statistically significant (*P* = .13) (Figure 1B).

Previous investigations have demonstrated that the area under the curve of docetaxel is associated with time to tumor progression in patients with non-small cell lung cancer (14). To exclude the possibility that the observed decreased survival time for subjects carrying the CYP1B1*3/*3 variant was the result of altered exposure to docetaxel, the pharmacokinetic data generated in a previous study (15) in 23 patients treated with docetaxel as a function of CYP1B1*3 genotype was analyzed. There was no statistically significant effect of genotype on the clearance of docetaxel (38.2 L/h, 95% confidence interval [CI] = 27.5 to 48.9 L/h, in patients who are homozygous or heterozygous for the wild-type allele; and 32.0 L/h, 95% CI = 26.9 to 37.0 L/h in patients who are homozygous for the variant allele) (P=.39, Mann-Whitney test). This is consistent with in vitro studies indicating that docetaxel is not directly metabolized by CYP1B1 (16).

Without wishing to be bound by any particular scientific theory, in light of previous investigations (2, 3, 5, 16), and the data obtained demonstrating no association between docetaxel pharmacokinetics and CYP1B1 genotype, this relationship is likely caused by alterations in cellular response to docetaxel caused by an indirect interaction. There is evidence, for example, that CYP1B1-mediated 17β-estradiol metabolites, such as quinonal and semiquinonal catechol estrogens, and methoxyestrogens, can bind tubulin, at the colchicine binding site (17, 18). These metabolites could be expected to interfere with the microtubule stabilizing effect of docetaxel, thus interfering with the efficacy of docetaxel treatment. This is further supported by the notion that CYP1B1-mediated estrogen metabolites that bind tubulin are more prevalent in the homozygote CYP1B1*3/*3 genotype (19).

### References

1. Bruno R, Hille D, Riva A, Vivier N, ten Bokkel Huinnink WW, van Oosterom AT, et al. Population pharmacokinetics/pharmacodynamics of docetaxel in phase II studies in patients with cancer. J Clin Oncol 1998;16(1):187-96.
2. McFadyen MC, McLeod HL, Jackson FC, Melvin WT, Doehmer J, Murray GI. Cytochrome P450 CYP1B1 protein expression: a novel mechanism of anticancer drug resistance. Biochem Pharmacol 2001;62(2):207-12.
3. Rochat B, Morsman JM, Murray GI, Figg WD, McLeod HL. Human CYP1B1 and anticancer agent metabolism: mechanism for tumor-specific drug inactivation? J Pharmacol Exp Ther 2001;296(2):537-41.
4. Carnell DM, Smith RE, Daley FM, Barber PR, Hoskin PJ, Wilson GD, et al. Target validation of cytochrome P450 CYP1B1 in prostate carcinoma with protein expression in associated hyperplastic and premalignant tissue. Int J Radiat Oncol Biol Phys 2004;58(2):500-9.
5. McFadyen MC, Cruickshank ME, Miller ID, McLeod HL, Melvin WT, Haites NE, et al. Cytochrome P450 CYP1B1 over-expression in primary and metastatic ovarian cancer. Br J Cancer 2001;85(2):242-6.
6. McFadyen MC, Breeman S, Payne S, Stirk C, Miller ID, Melvin WT, et al. Immunohistochemical localization of cytochrome P450 CYP1B1 in breast cancer with monoclonal antibodies specific for CYP1B1. J Histochem Cytochem 1999;47(11):1457-64.
7. Murray GI, Taylor MC, McFadyen MC, McKay JA, Greenlee WF, Burke MD, et al. Tumor-specific expression of cytochrome P450 CYP1B1. Cancer Res 1997;57(14):3026-31.
8. Hanna IH, Dawling S, Roodi N, Guengerich FP, Parl FF. Cytochrome P450 1B1 (CYP1B1) pharmacogenetics: association of polymorphisms with functional differences in estrogen hydroxylation activity. Cancer Res 2000;60(13):3440-4.
9. Landi MT, Bergen AW, Baccarelli A, Patterson DG, Jr., Grassman J. Ter-Minassian M, et al. CYP1A1 and CYP1B1 genotypes, haplotypes, and TCDD-induced gene expression in subjects from Seveso, Italy. Toxicology 2005;207(2):191-202.
10. Li KM, Todorovic R, Devanesan P, Higginbotham S, Kofeler H, Ramanathan R, et al. Metabolism and DNA binding studies of 4-hydroxyestradiol and estradiol-3,4-quinone in vitro and in female ACI rat mammary gland in vivo. Carcinogenesis 2004;25(2):289-97.
11. Shimada T, Watanabe J, Kawajiri K, Sutter TR, Guengerich FP, Gillam EM, et al. Catalytic properties of polymorphic human cytochrome P450 1B1 variants. Carcinogenesis 1999;20(8):1607-13.
12. Dahut WL, Gulley JL, Arlen PM, Liu Y, Fedenko KM, Steinberg SM, et al. Randomized phase II trial of docetaxel plus thalidomide in androgen-independent prostate cancer. J Clin Oncol 2004;22(13):2532-9.
13. Sasaki M, Tanaka Y, Kaneuchi M, Sakuragi N, Dahiya R. Alleles of polymorphic sites that correspond to hyperactive variants of CYP1B1 protein are significantly less frequent in Japanese as compared to American and German populations. Hum Mutat 2003;21(6):652.
14. Bruno R, Olivares R, Berille J, Chaikin P, Vivier N, Hammershaimb L, et al. Alpha-1-acid glycoprotein as an independent predictor for treatment effects and a prognostic factor of survival in patients with non-small cell lung cancer treated with docetaxel. Clin Cancer Res 2003;9(3):1077-82.
15. Louwerens M, Smorenburg C, Sparreboom A, Loos WJ, Verweij J, de Wit R. Phase I pharmacokinetic and sequence finding study of the combination of docetaxel and methotrexate in patients with solid tumours. Eur J Cancer 2002;38(4):497-504.
16. Bournique B, Lemarie A. Docetaxel (Taxotere) is not metabolized by recombinant human CYP1B1 in vitro, but acts as an effector of this isozyme. Drug Metab Dispos 2002;30(11):1149-52.
17. D'Amato RJ, Lin CM, Flynn E, Folkman J, Hamel E. 2-Methoxyestradiol, an endogenous mammalian metabolite, inhibits tubulin polymerization by interacting at the colchicine site. Proc Natl Acad Sci U S A 1994;91(9):3964-8,
18. Yager JD, Liehr JG. Molecular mechanisms of estrogen carcinogenesis. Annu Rev Pharmacol Toxicol 1996;36:203-32.
19. Li DN, Seidel A, Pritchard MP, Wolf CR, Friedberg T. Polymorphisms in P450 CYP1B1 affect the conversion of estradiol to the potentially carcinogenic metabolite 4-hydroxyestradiol. Pharmacogenetics 2000;10(4):343-53.
20. WO 01/58444 A (UNIV ABERDEEN [GB]; MURRAY GRAEME IAN [GB]; MELVIN WILLIAM THOMAS [GB]) 16 August 2001 (2001-08-16).

## Claims

1. A method of predicting responsiveness of a prostate tumor to a tubulin stabilization agent, wherein the method comprises:
determining a CYP1B1 genotype status of the tumor cell, wherein the genotype status at nucleotide position 4326 is determined; and
correlating homozygous variant CYP1B1 genotype status with unresponsiveness of the tumor cell to the tubulin stabilization agent, wherein the homozygous variant is associated with unresponsiveness of the tumor cell.

2. The method of claim 1, wherein the genotype status is determined by PCR methods, immunological methods, sequencing methods, expression level of CYP1B1, enzyme kinetics of CYP1B1.

3. The method of claim 1, wherein the CYP1B1 genotype status at nucleotide position 4326 is determined by one or more of sequencing methods, PCR methods, SNP Chip technology, or RFLP.

4. The method of claim 3, wherein PCR methods are one or more of real-time PCR, PCR, reverse transcriptase PCR, or allele-specific PCR.

5. The method of claim 1, wherein the CYP1B1 genotype status at amino acid position 432 is determined.

6. The method of claim 5, wherein the CYP1B1 genotype status at amino acid position 432 is determined by one or more of immunological methods or sequencing methods.

7. The method of claim 1, wherein a wildtype genotype status is L at amino acid position 432 and C at nucleotide position 4326.

8. The method of claim 7, wherein the heterozygous or homozygous variant CYP1B1 genotype status is V at amino acid position 432 and G at nucleotide position 4326.

9. The method of claim 1, wherein the tubulin stabilization agent is selected from one or more of docetaxel, paclitaxel, and derivatives thereof.

10. A method of selecting a subject having or at risk developing prostate cancer that will respond to docetaxel treatment, comprising:
detecting the presence or absence of a variation at nucleotide position 4326 or amino acid position 432 of CYP1B1, and
correlating an absence of a variation or the presence of a homozygous variation with an indication that the subject will not respond to docetaxel.

11. The method of claim10, wherein the detecting comprises PCR methods, immunological methods, sequencing methods, expression level of CYP1B1, or enzyme kinetics of CYP1B1.

12. Use of a kit in the method of claim 1, wherein the kit comprises:
oligonucleotide probes that differentiate the wild-type and variant alleles of CYP1B1, wherein the allele nucleotide position is 4326.

13. Use of a kit in the method of claim 1 according to claim 12, wherein the oligonucleotide probes are one or more of OLA or Taqman probes.

14. Use of a kit in the method of claim 1 according to claim 12, wherein the kit comprises:
oligonucleotide primres that amplify from about nucleotide 4300 to about nucleotide 4350 portion of CYP1B1.

15. Use of a kit in the method of claim 1 according to claim 12, wherein the kit comprises:
a microarray, at least one oligonucleotide primer that amplifies from about nucleotide 4300 to about nucleotide 4350 of CYP1B1.

## Patentansprüche

1. Verfahren zum Vorhersagen des Ansprechens eines Prostatatumors auf ein Tubulin-Stabilisierungsmittel, wobei das Verfahren umfasst:
Bestimmen eines CYP1 B1 Genotypstatus der Tumorzelle, wobei der Genotypstatus bei Nukleotidposition 4326 bestimmt wird; und
Korrelieren der homozygoten Variante des CYP1 B1 Genotypstatus mit Nichtansprechen der Tumorzelle auf das Tubulin-Stabilisierungsmittel, wobei die homozygote Variante mit Nichtansprechen der Tumorzelle in Zusammenhang steht.

2. Verfahren nach Anspruch 1, wobei der Genotypstatus durch PCR-Verfahren, immunologische Verfahren, Sequenzierungsverfahren, Expressionspegel von CYP1 B1, Enzymkinetik von CYP1 B1 bestimmt wird.

3. Verfahren nach Anspruch 1, wobei der CYP1 B1 Genotypstatus bei Nukleotidposition 4326 durch eines oder mehrere von Sequenzierungsverfahren, PCR-Verfahren, SNP Chiptechnik oder RFLP bestimmt wird.

4. Verfahren nach Anspruch 3, wobei die PCR-Verfahren eines oder mehrere sind von Echtzeit-PCR, PCR, reverse Transkriptase PCR oder allelspezifische PCR.

5. Verfahren nach Anspruch 1, wobei der CYP1 B1 Genotypstatus bei Aminosäureposition 432 bestimmt wird.

6. Verfahren nach Anspruch 5, wobei der CYP1 B1 Genotypstatus bei Aminosäureposition 432 durch eines oder mehrere von immunologischen Verfahren oder Sequenzierungsverfahren bestimmt wird.

7. Verfahren nach Anspruch 1, wobei ein Wildtyp-Genotypstatus bei Aminosäureposition 432 L ist und bei Nukleotidposition 4326 C ist.

8. Verfahren nach Anspruch 7, wobei die heterozygote oder homozygote Variante des CYP1 B1 Genotypstatus bei Aminosäureposition 432 V ist und bei Nukleotidposition 4326 G ist.

9. Verfahren nach Anspruch 1, wobei das Tubulin-Stabilisierungsmittel aus einem oder mehreren von Docetaxel, Paclitaxel und Derivaten davon ausgewählt wird.

10. Verfahren zum Auswählen eines Probanden, der Prostatakrebs hat oder bei dem das Risiko besteht, dass er Prostatakrebs entwickelt, der auf Behandlung mit Docetaxel ansprechen wird, wobei das Verfahren umfasst:
Feststellen des Vorhandenseins oder der Abwesenheit einer Variation bei Nukleotidposition 4326 oder Aminosäureposition 432 von CYP1B1, und
Korrelieren einer Abwesenheit einer Variation oder der Anwesenheit einer homozygoten Variation mit einem Anzeichen dafür, dass der Proband nicht auf Docetaxel ansprechen wird.

11. Verfahren nach Anspruch 10, wobei das Feststellen PCR-Verfahren, immunologische Verfahren, Sequenzierungsverfahren, Expressionspegel von CYP1 B1 oder Enzymkinetik von CYP1 B1 umfasst.

12. Verwendung einer Ausrüstung bei dem Verfahren von Anspruch 1, wobei die Ausrüstung aufweist:
Oligonukleotidsonden, die den Wildtyp und variante Allele von CYP1 B1 unterscheiden, wobei die Allel-Nukleotidposition 4326 ist.

13. Verwendung einer Ausrüstung bei dem Verfahren von Anspruch 1 nach Anspruch 12, wobei die Oligonukleotidsonden eine oder mehrere sind von OLA- oder Taqman-Sonden.

14. Verwendung einer Ausrüstung bei dem Verfahren von Anspruch 1 nach Anspruch 12, wobei die Ausrüstung aufweist:
Oligonukleotid-Primer, die sich von ca. Nukleotid 4300 Anteil bis zu ca. Nukleotid 4350 Anteil von CYP1 B1 verstärken.

15. Verwendung einer Ausrüstung bei dem Verfahren von Anspruch 1 nach Anspruch 12, wobei die Ausrüstung aufweist:
einen Mikroarray, wenigstens einen Oligonukleotid-Primer, der sich von ca. Nukleotid 4300 bis zu ca. Nukleotid 4350 von CYP1 B1 verstärkt.

## Revendications

1. Procédé de prédiction de la faculté de réponse d'une tumeur de la prostate à un agent de stabilisation de la tubuline, dans lequel le procédé comprend :
la détermination d'un statut du génotype de CYP1 B1 de la cellule tumorale, dans laquelle le statut du génotype à la position nucléotidique 4326 est déterminé ; et
la corrélation du statut du génotype de CYP1 B1 variant homozygote avec l'absence de réponse de la cellule tumorale à l'agent de stabilisation de la tubuline, dans laquelle le variant homozygote est associé à l'absence de réponse de la cellule tumorale.

2. Procédé selon la revendication 1, dans lequel le statut du génotype est déterminé par des procédés de PCR, des procédés immunologiques, des procédés de séquençage, le niveau d'expression de CYP1 B1, la cinétique enzymatique de CYP1B1.

3. Procédé selon la revendication 1, dans lequel le statut du génotype de CYP1 B1 à la position nucléotidique 4326 est déterminé par un ou plusieurs parmi les procédés de séquençage, les procédés de PCR, la technologie de puce SNP, ou le RFLP.

4. Procédé selon la revendication 3, dans lequel les procédés de PCR sont un ou plusieurs parmi une PCR en temps réel, une PCR, une transcriptase inverse PCR ou une PCR spécifique d'allèle.

5. Procédé selon la revendication 1, dans lequel le statut du génotype de CYP1 B1 à la position d'acide aminé 432 est déterminé.

6. Procédé selon la revendication 5, dans lequel le statut du génotype de CYP1 B1 à la position d'acide aminé 432 est déterminé par un ou plusieurs parmi des procédés immunologiques ou des procédés de séquençage.

7. Procédé selon la revendication 1, dans lequel un statut du génotype de type sauvage est L à la position d'acide aminé 432 et C à la position nucléotidique 4326.

8. Procédé selon la revendication 7, dans lequel le statut du génotype de CYP1B1 variant hétérozygote ou homozygote est V à la position d'acide aminé 432 et G à la position nucléotidique 4326.

9. Procédé selon la revendication 1, dans lequel l'agent de stabilisation de la tubuline est choisi parmi un ou plusieurs du docétaxel, du paclitaxel et des dérivés de ceux-ci.

10. Procédé de sélection d'un sujet ayant ou à risque de développer un cancer de la prostate qui répondra à un traitement par le docétaxel, comprenant :
la détection de la présence ou de l'absence d'une variation à la position nucléotidique 4326 ou à la position d'acide aminé 432 de CYP1 B1, et
la corrélation d'une absence d'une variation ou de la présence d'une variation homozygote avec une indication que le sujet ne répondra pas au docétaxel.

11. Procédé selon la revendication 10, dans lequel la détection comprend des procédés de PCR, des procédés immunologiques, des procédés de séquençage, le niveau d'expression de CYP1 B1 ou la cinétique enzymatique de CYP1B1.

12. Utilisation d'un kit dans le procédé selon la revendication 1, dans laquelle le kit comprend :
des sondes oligonucléotidiques qui différencient les allèles de type sauvage et variants de CYP1 B1, où la position allélique du nucléotide est 4326.

13. Utilisation d'un kit dans le procédé de la revendication 1 selon la revendication 12, dans laquelle les sondes oligonucléotidiques sont une ou plusieurs parmi les sondes OLA ou Taqman.

14. Utilisation d'un kit dans le procédé de la revendication 1 selon la revendication 12, dans laquelle le kit comprend :
des amorces oligonucléotides qui amplifient la partie d'environ le nucléotide 4300 à environ le nucléotide 4350 de CYP1B1.

15. Utilisation d'un kit dans le procédé de la revendication 1 selon la revendication 12, dans laquelle le kit comprend :
un micro-réseau, au moins une amorce oligonucléotidique qui amplifie d'environ le nucléotide 4300 à environ le nucléotide 4350 de CYP1 B1.
